Europäisches Patentamt

(19) European Patent Office

Office européen des brevets

(11) **EP 0 398 924 B1**

(12) **EUROPEAN PATENT SPECIFICATION**

(45) Date of publication and mention
of the grant of the patent:
**26.06.1996 Bulletin 1996/26**

(21) Application number: **89901783.4**

(22) Date of filing: **11.01.1989**

(51) Int. Cl.[6]: **A61K 45/00**, A61K 38/00,
C12P 21/00, C07K 2/00

(86) International application number:
**PCT/US89/00104**

(87) International publication number:
**WO 89/06545 (27.07.1989 Gazette 1989/16)**

(54) **HISTAMINE RELEASE INHIBITORY FACTOR AND PREPARATIONS THEREFORE**

DIE FREISETZUNG DES HISTAMIN HEMMENDEN FAKTORS SOWIE DIESEN ENTHALTENDE PRÄPARATE

FACTEUR INHIBITANT LA LIBERATION D'HISTAMINE ET PREPARATIONS LE CONTENANT

(84) Designated Contracting States:
**AT BE CH DE FR GB IT LI LU NL SE**

(30) Priority: **12.01.1988 US 143094**

(43) Date of publication of application:
**28.11.1990 Bulletin 1990/48**

(73) Proprietor: **BOARD OF REGENTS THE UNIVERSITY OF TEXAS SYSTEM Austin, Texas 78701 (US)**

(72) Inventors:
• **ALAM, Rafeul**
  **Galveston, TX 77554 (US)**
• **GRANT, J., Andrew**
  **Galveston, TX 77551 (US)**
• **LETT-BROWN, Michael, A.**
  **Galveston, TX 77550 (US)**

(74) Representative: **Dost, Wolfgang, Dr.rer.nat., Dipl.-Chem. et al
Patent- und Rechtsanwälte
Bardehle . Pagenberg . Dost . Altenburg .
Frohwitter . Geissler & Partner
Postfach 86 06 20
81633 München (DE)**

(56) References cited:
• **Fed.Proc., Vol. 46, 1987.R Alam, D.M: Lewis and S.A. Olenchock:"Induction of histamine releasing factor(HRF) synthesis in guinea-pig:simultaneous production of a histamine release inhibitory factor(HRIF) and an inhibitor of HRFsynthesis(IHS).", see page 928**
• **Journal of clinical investigation, Vol. 82, 1988 Refeul Alam et al: "Identifica tion of a Histamine Release Inhibitory Factor Produced by Human Mononclear Cell s in Vitro. ", se page 2056 - page 2062 See especially abstract, materials and**
• **Journal of allergy and clinical immunology. , Vol. 79, No. 1, 1987 Rafeul Alam et al: "The magnitude of the spontaneous production of histamine-releasing factor (HRF) by lymphocytes in vitro correlates with the state of bronchial hyperreactivity in patients with asth", see page 103 - page 108**
• **Federation proceedings., Vol.45, No. 11, 1986 J. Andrew Grant et al:"Activation of basophils. ", see page 2653 - page 2658**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

A. FIELD OF THE INVENTION

The present invention relates to a novel inhibitor of allergic reactions, designated histamine release inhibitory factor (HRIF). This factor inhibits release of allergic mediators from basophils and mast cells triggered by histamine releasing factors (HRF) now believed to play a key role in the development of asthma and other allergic disorders. This factor is likely to prove useful as an inhibitor of allergic disorders and other human diseases in which basophils and mast cells may have a pathologic role.

B. DESCRIPTION OF THE RELATED ART

Basophils and mast cells have been the subjects of scientific investigation since they were described by Paul Ehrlich in the 1870s. Mast cells are generally found in connective tissue; basophils, in the blood. The two cell types have many similarities. Both contain numerous metachromatically staining granules, both have cell surface receptors that bind IgE with high affinity, and both contain a myriad of diverse allergic mediators that can cause symptoms ranging from itching of the skin to the most life-threatening clinical situation, anaphylaxis. Basophils and mast cells collectively account for virtually all of the total body histamine and are collectively referred to as histamine-containing proallergic cells.

There is convincing evidence that mast cells and basophils are essential for induction of allergic hypersensitivity reactions. For example, substantial evidence suggests that mast cells are the primary effector cell of such allergic diseases as asthma, allergic rhinitis; conjunctivitis, urticaria and anaphylaxis. Increased numbers of mast cells have been found in bronchoalveolar lavage fluid, respiratory mucosa, nasal mucosa, and biopsy specimens of urticarial lesions in these disorders. In addition, mast cell mediators, such as histamine and prostaglandin (PG) $D_2$, have been recovered from serum, bronchoalveolar lavage fluid, nasal washings, and skin blister fluid during natural and provoked allergic reactions. More recently, the mast cell granule-specific enzyme tryptase (not found in basophils) has been detected in serum from patients with allergic and anaphylactoid reactions. Finally, most mast cell-derived mediators, alone or in combination, can evoke such typical allergic symptoms as bronchospasm, angioedema, cough, mucus secretion, rhinorrhea, sneezing, and wheal-and-flare skin reactions and mast cell-specific degranulating agents can induce allergic reactions in vivo.

In addition, histologic evidence suggests that mast cells are involved in the pathogenesis of a number of chronic inflammatory diseases. Increased numbers of mast cells have been detected in biopsy specimens from patients with rheumatoid arthritis, ulcerative colitis, Crohn's disease, sarcoidosis, hypersensitivity pneumonitis, pulmonary fibrosis, nasal polyps, atopic dermatitis, allergic contact dermatitis, bullous pemphigoid, keloid formation, scleroderma and progressive systemic sclerosis, acute and chronic graft vs. host disease, and parasitic infestation. Mast cells also have been implicated in regulation of nerve growth, and regulation of mast cell degranulation has proved useful in neurofibromatosis. Furthermore, increased levels of histamine were detected in the bronchoalveolar lavage fluid from patients with sarcoidosis, hypersensitivity pneumonitis, and idiopathic pulmonary fibrosis. Cromolyn sodium, a putative mast cell stabilizer, has been found beneficial to a subgroup of patients with ulcerative colitis, particularly those with proctitis.

Basophils appear to be particularly involved in some forms of allergic contact dermatitis, especially to poison ivy and, experimentally, to dinitrochlorobenzene. These cells represent 5% to 15% of the total infiltrating cells and are present within eight hours after application of the allergen to the skin. Given the number of inflammatory mediators released by mast cells and basophils, it is conceivable that sustained piecemeal degranulation of these cells contributes to the chronic inflammatory nature of the aforementioned disorders.

Unfortunately, despite the convincing evidence linking mast cells and basophils to these and other disease states, the precise pathogenesis of mast cell and basophil dependent disorders, including allergic disease, is incompletely understood. For example, it is known that mast cells and basophils are stimulated to release histamine, leukotrienes, and other inflammatory mediators by the bridging of cell surface-bound IgE antibodies by appropriate allergens (or anti-IgE antibodies), but the severity of a number of allergic diseases, for example bronchial asthma, rhinitis, and conjunctivitis, does not correlate with a patient's IgE level. Moreover, although mast cells are believed to play a role in various other diseases such as inflammatory bowel disease, rheumatoid arthritis, pulmonary fibrosis, and sarcoidosis, in the majority of these diseases, IgE antibody cannot be found.

Therefore, it appears that other mechanisms of allergic mediator release play a critical role in pathogenesis of allergic diseases and other disorders mentioned above in which basophils and mast cells have been implicated. Elucidation of such mechanisms has been and remains the goal of many skilled medical scientists.

One of the most exciting developments in this area was the discovery of histamine releasing activity (HRA), cytokines designated herein as histamine releasing factor(s) (HRF), by investigators working in the laboratories of the present inventors. Thueson, et al., (J. Immunol., 123:626 (1979); J. Immunol., 123:633 (1979)) and Lett-Brown, et al., (Cell Immunol., 87:434 (1984); Cell Immunol., 87:445 (1984)) first reported that antigen or mitogen stimulated human mononuclear cells secrete a proteinaceous factor that induces release of histamine from basophils and mast cells. Other laboratories then confirmed the synthesis of HRF by mononuclear cells. It has now been shown that HRF is also synthesized by B-lymphocytes and T-lymphocytes, alveolar macrophages, platelets, neutrophils, and blood monocytes cultured in vitro. The wide variety of cell types reported to secrete HRF suggests that it has considerable biologic importance. In addition to mediating histamine release, HRF has been shown to induce secretion of leukotrienes and to be chemotactic for basophils and monocytes. (For a review, see Grant, et al., Fed. Proc., 45:2653 (1986), J. Allergy Clin. Immunol., 77:407 (1986), and Alam, Insights in Allergy, Vol. 2, no. 6 (1987), CV Mosby, St. Louis, all incorporated herein by reference.)

Numerous studies provide data directly supporting the importance of HRF as a mediator of human allergic disease. For example, an HRF-like material has been obtained from skin blister fluid obtained during the late allergic reaction, now considered an important factor in the pathogenesis of chronic asthma and other allergic conditions. HRF has also been recovered in nasal washings. In addition, HRF induces bronchoconstriction on inhalation by asthmatic subjects and a wheal-and-flare reaction in humans and non-human primates. Mononuclear cells from asthmatic patients have been shown to spontaneously produce relatively large amounts of HRF, and HRF production is enhanced on in vitro incubation with specific allergen. Moreover, the magnitude of spontaneous HRF production correlates with the severity of bronchial hyperreactivity in asthmatic patients [Alam, et al., J. Allergy Clin. Immunol., 79:103 (1987)]. These findings suggest that in asthmatic patients, increased spontaneous HRF production may cause a sustained release of allergic mediators from mast cells or basophils, resulting in chronic inflammation and ultimately leading to the development of bronchial hyperreactivity.

In addition, although immunotherapy is a well accepted modality for treating allergic diseases, the mechanism of its action is still obscure. Changes in serum IgE antibody do not correlate well with the efficacy of immunotherapy. Serum IgG blocking antibody usually rises after prolonged treatment. Although some investigators have shown a correlation between the efficacy of immunotherapy and the level of IgG antibody, the correlation is often too tenuous to imply a casual relationship. Recently, however, one of the present inventors has discovered that immunotherapy abrogates the seasonal rise in HRF production and diminishes spontaneous HRF production in patients with clinical improvements. Thus, there is a high correlation between symptom-medication score and spontaneous HRF production (r=0.92, p=0.0002).

Given the important role played by HRF in the pathogenesis of allergic diseases and other disorders including the release of mediators from basophils and mast cells, it is likely that an agent capable of inhibiting HRF-induced mediator release could provide a valuable tool in treating mast cell/basophil-dependent disorders, which include the allergic diseases. A guinea pig inhibitor of histamine release has been reported (Alam et al., Fed. Proc. 46 (1987), page 928). However, despite the need for such an agent, no endogenous substance capable of inhibiting HRF induced histamine release from human mast cells and basophils has heretofore been described. Fortunately, the present inventors have now discovered a histamine release inhibitory factor having the desired properties. This factor, its production and properties, are discussed in the present disclosure.

## SUMMARY OF THE INVENTION

It is a general object of the present invention to provide an inhibitor of histamine releasing factor. It is also an object of the present invention to provide a specific factor capable of inhibiting release of histamine from mast cells or basophils triggered with histamine releasing factor. It is also an object of the present invention to provide methods for producing such histamine release inhibitory factors, cell lines capable of producing such factors, and to describe the substantial purification of this factor.

Therefore, the invention is directed to a therapeutic composition comprising a pharmaceutically acceptable carrier and a substantially purified histamine release inhibitory factor wherein the factor comprises a substantially purified human histamine release inhibitory factor (HRIF) having the following characteristics: a molecular weight of about 8,000-10,000 daltons as determined by gel exclusion chromatography; lability upon treatment with trypsin or chymotrypsin; stability when heated at 60°C. for one hour; and stability when treated with neuraminidase.

The invention further provides a composition comprising a substantially purified proteinaceous factor, said factor exhibiting biologic properties that include inhibition of allergic mediator release from human basophils or human mast cells triggered with histamine releasing factor. A composition that includes this factor may be further characterized by physicochemical and biologic characteristics. For example, the factor may be further defined as having molecular weight of about 8,000-10,000 daltons as determined by gel exclusion chromatography, or, as binding to DEAE cellulose equilibrated with about 1 mM phosphate buffer at about pH 7.4, and being eluted with about 100 mM to 200 mM NaCl at about pH 7.4. In addition, the factor may be characterized by its stability or lability to various treatments. For example, in various embodiments the factor is defined as having biologic properties including lability of allergic mediator release inhibitory activity upon treatment with trypsin or chymotrypsin, or as having biologic properties including stability of allergic mediator release inhibitory activity when treated with neuraminidase, or when heated at 60°C. for one hour.

Finally, the factor may also be defined as being retained by spectrapor-3 dialysis tubing having a molecular weight cut-off of about 3,500 daltons.

Additionally, the factor may be defined by its pattern or specificity of inhibitory activity when basophils or mast cells are triggered with particular agonists or secretagogues known to induce allergic mediator release under conditions known to those of skill in the art. Of course, the factor is generally defined as being capable of inhibiting mediator release from cells triggered with HRF. In addition, the factor may be more particularly defined as not inhibiting allergic mediator release from cells triggered with allergen, antibodies specific for IgE, concanavalin A, phorbol myristate acetate, or the anaphylatoxin C5a.

An additional specific embodiment is directed toward inhibition of release of a particular allergic mediator, histamine. As those of skill in the art are aware, basophils and mast cells contain a number of other allergic mediators, such as leukotrienes. The term "allergic mediators" is intended to encompass these compounds. However, the experiments described herein have been performed using histamine as a prototypic mediator. Therefore, a specific embodiment is directed towards a composition specifically characterized as inhibiting histamine release.

The invention also comprises a substantially purified human protein factor capable of inhibiting histamine release from human proallergic cells triggered with histamine releasing factor, said protein factor having a molecular weight from 20.000 to 30.000 Da. The invention also comprises a substantially purified proteinaceous factor exhibiting biologic properties which include inhibition of allergic mediator release from human basophils or human mast cells triggered with histamine releasing factor, wherein said factor is obtained by the process of preparing a human mononuclear cell conditioned supernatant, fractionating the supernatant to provide a fraction which includes the factor in substantially purified form, and collecting the fraction. In a more particular embodiment, the conditioned cell supernatant is prepared by obtaining a mononuclear cell fraction of leukocytes from an individual, culturing the mononuclear cell fraction in an appropriate medium to produce a conditioned supernatant, and separating the supernatant from the cultured cells. In an even more particular embodiment, the leukocytes are obtained from peripheral blood. However, it is believed that leukocytes from other sources, such as spleen cells, thoracic duct cells, or lymph-node cells, including cells obtained from tonsillar lymph nodes, may be used. Of course, where peripheral blood is used as a leukocyte source, fractionation, for example, by centrifugation over a Ficoll-Hypaque gradient, may be necessary to obtain the mononuclear fraction.

In an additional preferred embodiment, the conditioned supernatant is fractionated into protein fractions of selected molecular weight ranges to provide a fraction which includes the protein in substantially purified form. And, in a yet further embodiment, the fraction is selected by assaying various fractions for the ability to inhibit histamine release from human basophils or human mast cells triggered with histamine releasing factor. Finally, in additional highly preferred embodiments, the mononuclear cells are cultured in a tissue culture medium containing histamine, and the factor is defined as a human factor. In addition, the factor is also synthesized by human mononuclear cells cultured with mitogens such as concanavalin A or with antigens causing human allergic sensitivity, such as ragweed pollen or dust mite allergenic extracts.

The invention also encompasses compositions comprising substantially purified proteinaceous factors corresponding biologically to histamine release inhibitory factor produced by cell line JW-RW. Of course, as those of skill in the art will appreciate, a factor that corresponds biologically to the histamine release inhibitory factor produced by a certain cell line need not actually be produced by that line; it need only have the relevant biologic activities of the factor produced by the line. Therefore, in an additional embodiment, the composition comprises a substantially purified proteinaceous histamine release inhibitory factor actually produced by cell line JW-RW. In addition, the invention encompasses cell lines producing histamine release inhibitory factor corresponding to that produced by cell line JW-RW, and to a human cell line JW-RW.

The invention also provides a method for inhibiting release of an allergic mediator from human histamine containing proallergic cells by exposing such cells to the proteinaceous factor described above. In a more specific embodiment, the allergic mediator is specifically defined as being histamine.

In addition, the invention provides anti-allergic preparations, and preparations useful in the treatment of other disorders in which the release of mediators from basophils and mast cells is causal of pathologic symptomatology. In general, these preparations contain a pharmaceutically acceptable carrier and a substantially purified histamine release inhibitory factor. Methods for treating allergic individuals and others with illnesses caused by activation of basophils and mast cells comprising administering to such individuals a therapeutically acceptable dosage of the preparation are also provided as specific embodiments.

The invention also encompasses methods for producing histamine release inhibitory factor and for producing human histamine release inhibitory factor-producing cell lines. In general, the first method comprises preparing a human mononuclear cell conditioned supernatant and assaying it for the histamine release inhibitory activity. Of course, in a more preferred embodiment, the culture medium contains histamine, preferably at concentration from about $10^{-6}$ M to $10^{-10}$ M, but histamine is substantially removed from the conditioned supernatant prior to assay. In yet a further embodiment, the fractionated supernatant is assayed on a cell suspension containing human basophils or human mast cells exposed to histamine releasing factor.

The second method, directed to the generation of histamine release inhibitory factor producing cell lines, comprises obtaining leukocytes from an individual, culturing the cells in a suitable tissue culture medium containing an antigen, maintaining the cells in a culture medium containing interleukin-2 and feeder cells, and assaying the cultured cells for the ability to produce a histamine release inhibitory factor. In a preferred embodiment, the individual that serves as the leukocyte source will be an individual undergoing immunotherapy for an allergic disorder and the allergen added to the culture will correspond to an antigenic component of the preparation used for immunotherapy. In another preferred embodiment, the cells used to start the cell line will be enriched for antigen-binding T cells prior to placing in culture.

Finally, the invention also encompasses clinical assays adapted for monitoring or diagnosing individuals. One such assay comprises obtaining a mononuclear cell fraction of leukocytes from an individual and culturing the mononuclear cell fraction in a suitable culture medium to produce a conditioned supernatant. The conditioned supernatant is then assayed for histamine release inhibitory activity. Of course, in the clinical setting, it will often be desirable to compare the amount of histamine release inhibitor produced by the patient cells to a standard preparation containing a known amount of histamine release inhibitory activity. In general, it is believed that this assay will be most useful in monitoring patients with allergic diseases, particularly patients undergoing immunotherapy. However, the assay is likely to find considerable utility in monitoring all disease states wherein allergic mediator release from basophils or mast cells is associated with pathogenesis of the disease.

The invention also provides a clinical assay wherein biological fluids such as whole blood or components, respiratory secretions or washings, urine, joint fluid, and other body fluids are assayed for the presence of histamine release inhibitory activity. It is believed that absolute levels of the factor in these fluids will correlate with disease activity.

The above noted and other objects and advantages of the present invention will become more apparent from the detailed description of the preferred embodiments described below.

## BRIEF DESCRIPTION OF THE DRAWINGS

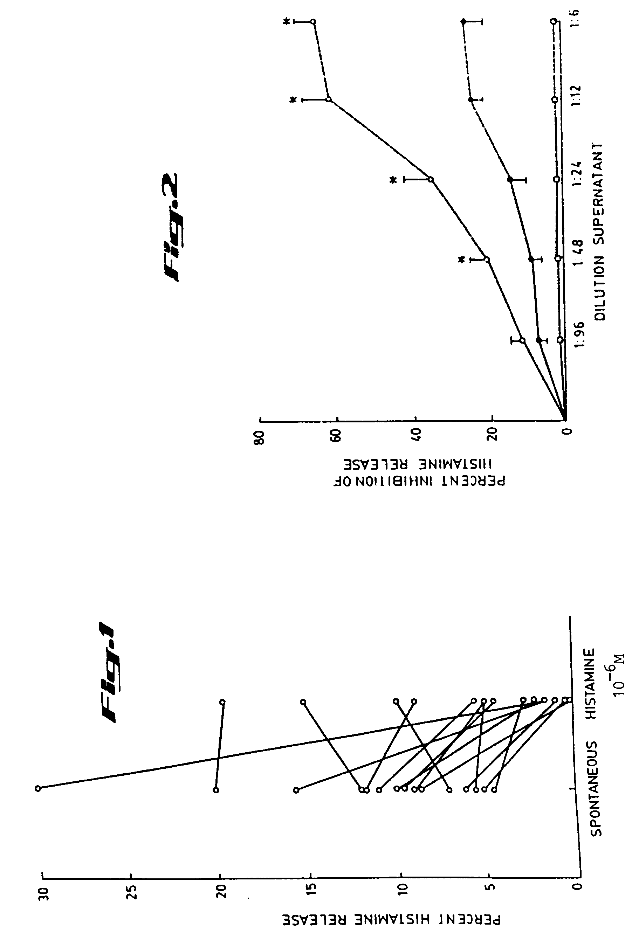

FIG. 1 -- Effect of culture of mononuclear cells with histamine upon the activity of HRF of the supernatants. Mononuclear cells were from 15 healthy donors which were cultured alone or in the presence of histamine ($10^{-6}$ M) for 24 hours. Supernatants were extensively dialyzed to remove histamine and tested for histamine releasing activity in the basophil histamine release test. Unstimulated mononuclear cell supernatant showed HRF activity and this activity was suppressed in 12 supernatants then the cells were cultured with histamine. This suggested that histamine might reduce the synthesis HRF or preferentially stimulate the production of an inhibitor.

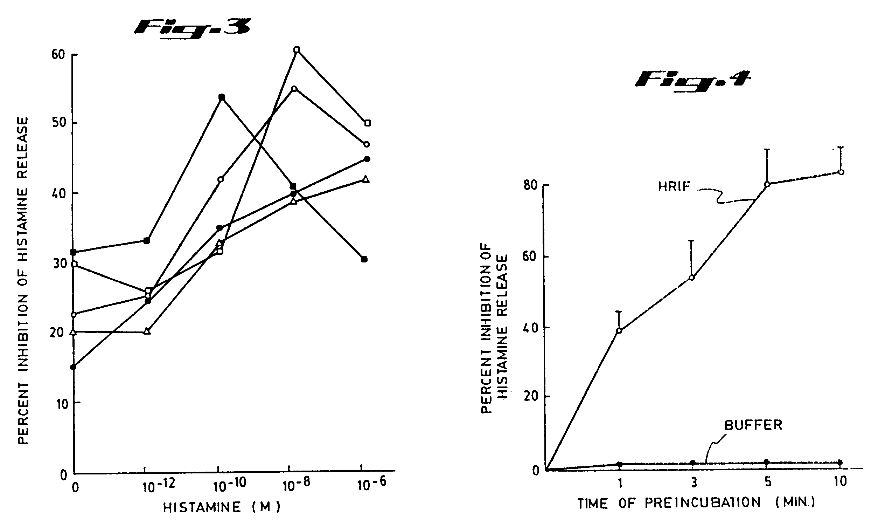

FIG. 2 -- Production of HRIF by human mononuclear cells (MNC). MNC from six healthy donors were cultured in RPMI either alone (●----●) or in the presence of histamine ($10^{-8}$ M) (o----o) for 24 hours. Culture supernatant was harvested by centrifugation, dialyzed twice against 1000 volumes of water and 100 volumes of HCM buffer in order to remove histamine and then tested for inhibition of HRF-induced histamine release from basophils obtained from three allergic donors. HRF-induced histamine release ranged from 25-44%. Data are expressed as X ± S.E.M. Unstimulated mononuclear cells cultured in RPMI spontaneously produced some HRIF and this production was significantly enhanced in the presence of histamine (*:P<0.001, t test). Histamine cultured without MNC (□----□) had no inhibitory activity. This experiment was repeated with mononuclear cells from 10 donors but using a narrower range of dilutions. Similar results were obtained: inhibition of histamine release by histamine-stimulated supernatants ranged from 40-80%.

FIG. 3 -- Dose-dependent production of HRIF by mononuclear cells from five healthy donors stimulated with various concentrations of histamine. Dialyzed supernatants (1:6 final dilution) were tested for inhibition of HRF-induced histamine release from basophils obtained from two allergic donors. HRF-induced histamine release ranged from 33-40%.

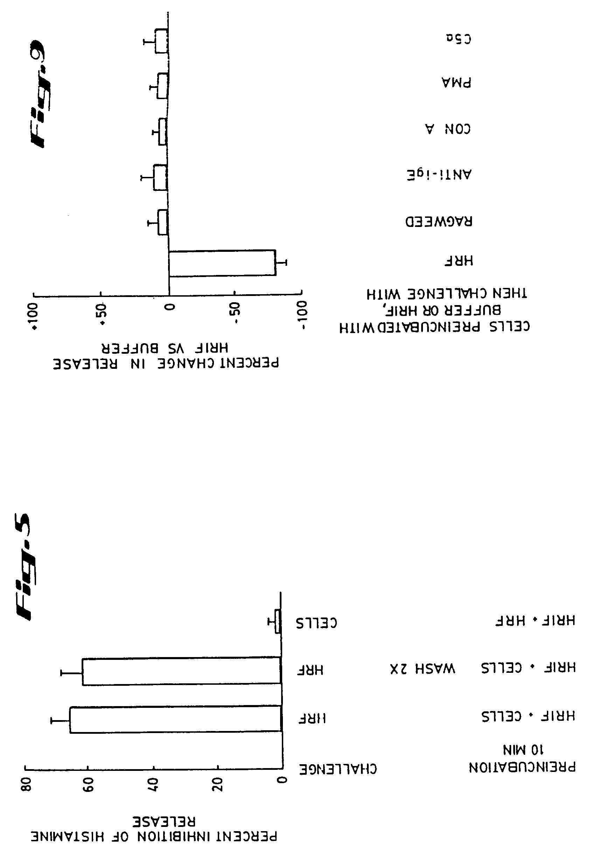

FIG. 4 -- Effect of preincubation period of cells upon the activity of HRIF. Basophil leukocytes were preincubated with HRIF (fifteen fold concentrated histamine stimulated supernatant), (o----o) for 40-60 seconds, 3, 5, and 10 minutes at room temperature and then challenged with HRF. Buffer (●----●) was used in the control experiments. Maximum inhibition was seen when the cells were preincubated with HRIF for five minutes or more. Results are X± S.E.M of six experiments. Control histamine release ranged from 18-30%.

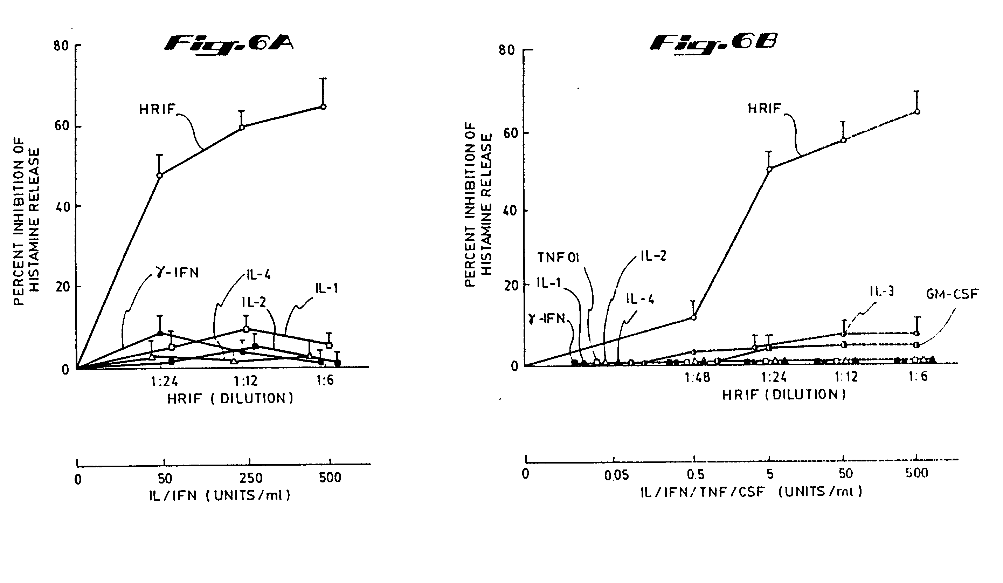

FIG. 5 -- Effect of removal of HRIF after preincubation upon HRF-induced histamine release from basophils. Leukocytes were preincubated with HRIF for ten minutes then centrifuged and washed twice against 40 volumes of buffer and then challenged with HRF. The inhibition of histamine release was compared with HRIF-treated cells that were not washed prior to adding HRF. Controls (not shown) included preincubation of cells in buffer followed by washing and HRF challenge. The percentage of histamine release was identical to cells that were not washed.

In another set of experiments, HRIF was preincubated with HRF for ten minutes and then cells were added. There was no inhibition of histamine release in these experiments. Results (X± S.E.M) of six experiments are shown. Control histamine release by HRF was in the range of 20-38%.

FIG. 6A -- Histamine release inhibitory activity of recombinant interleukins 1, 2, and 4 and gamma-interferon. Leukocytes were separately preincubated with HRIF, interleukins and interferon (50, 250, and 500 units per ml) for five minutes and then challenged with HRF. Results (X±S.E.M) of six experiments (for gamma-interferon n=4) are shown.

None of these cytokines inhibited HRF-induced histamine release from basophils. Control histamine release by HRF ranged from 20-34%.

FIG. 6B -- Leukocytes were isolated from venous blood obtained from 6 normal subjects and preincubated with five log concentrations of IL 1, 2, 3 and 4, tumor necrosis factor, GM-colony stimulating factor and gamma-interferon for 5 min. The cells were then challenged with a batch of HRF and incubated for another 30 min. and then analyzed for histamine release. In the control experiments, leukocytes were preincubated with HRIF-containing MNC-supernatant or buffer. The control histamine release by HRF in the presence of buffer was in the range of 25-55%.

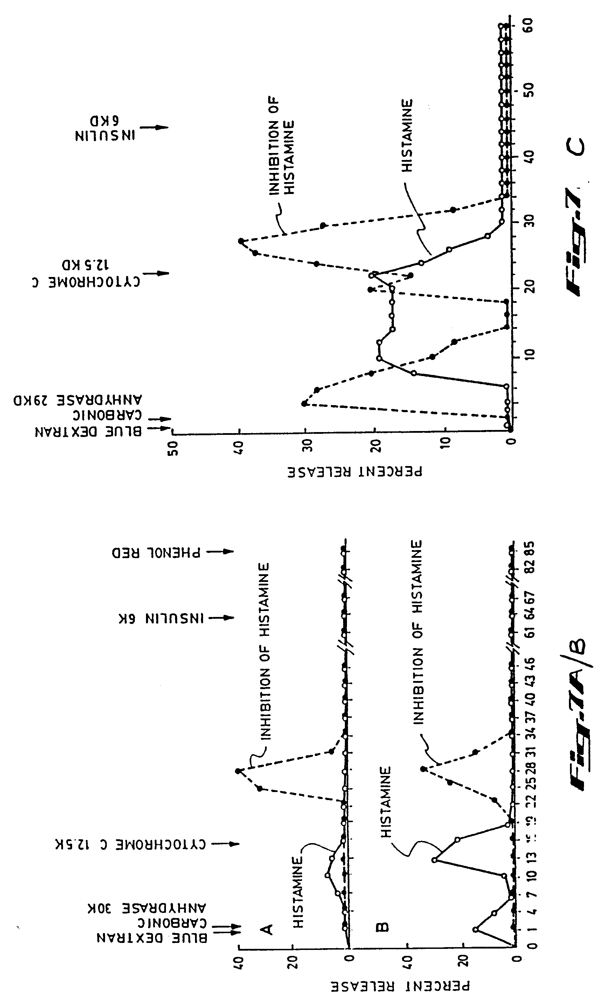

FIG. 7 -- Sephadex G-50 superfine gel chromatography of histamine-(7A) and concanavalin A-stimulated (7B and C) MNC supernatant. For FIG. 7A and 7B, fifteen fold concentrated supernatant (2 ml) was applied to a column (90 cm X 1.5 cm) that was precalibrated with marker proteins. One milliliter fractions were collected and every third fraction was tested at a dilution 1:3 for inhibition of HRF-induced HR from basophils. HRIF activity ($\bullet$----$\bullet$) of histamine-stimulated supernatant appeared in the fractions which corresponded to the MW range 8K-10K. A small peak of HRF (o—o) was found in the 15K-30K range. This experiment was repeated twice and a similar elution profile of HRIF was noted. The inventors also performed gel filtration of concanavalin A-stimulated mononuclear cell supernatant from healthy donors. HRIF activity was detected in the region which corresponded to MW 8K-10K whereas HRF activity appeared in the void volume (MW >30K) and in the MW range 15K-30K. This experiment was repeated twice and similar results were obtained. Control histamine release ranged from 26-48%.

FIG. 7C -- Twenty-fold concentrated supernatant (2 ml) was applied to a column (90 cm x 1.5 cm) that was precalibrated with marker proteins. One milliliter fractions were collected, and every other fraction was tested for direct histamine release (o) and for the inhibition of HRF-induced histamine release ($\bullet$) from basophils. This profile of HRIF activity has been observed in 1 of 3 MNC donors.

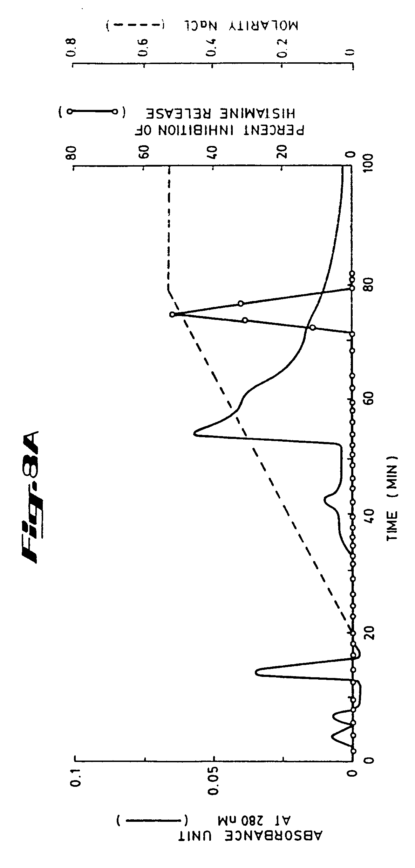

FIG. 8 -- Chromatography of concanavalin A-stimulated MNC supernatant on DEAE (DE-52) cellulose. Supernatant fluid was dialyzed against 1 mM phosphate buffer, pH 7.4. Approximately 1.0 gm of dry gel was combined with 10 ml of dialyzed supernatant and gently rocked at 4°C. for 15 hours. A column was packed with the gel and two volumes of the same buffer passed through the column. Subsequently the column was eluted with a linear gradient from 1 mM to 500 mM NaCl in 1 mM phosphate buffer, pH 7.4. The total volume of the gradient was about 40 ml and 2 ml fractions were collected. Fractions were dialyzed against HCM buffer and assayed for HRIF. HRIF was eluted from the column at approximately 100 mM NaCl.

FIG. 8A -- Anion exchange high performance liquid Chromatography of HRIF.

FIG. 8B -- Size exclusion HPLC of MNC-derived HRIF.

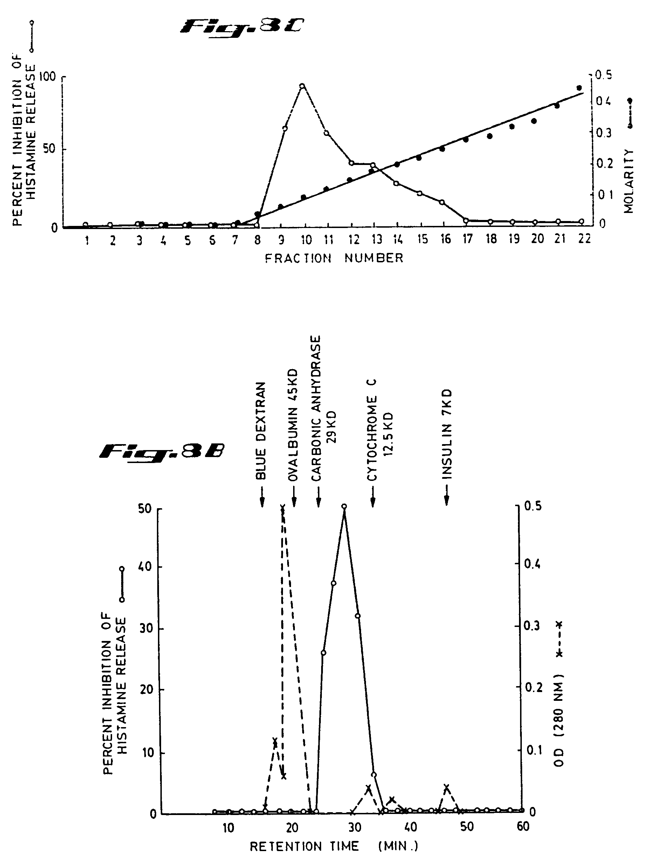

FIG. 9 -- Effect of histamine stimulated-mononuclear cell supernatants upon histamine release by HRF, ragweed allergen ($10^{-6}$ w/v), anti-IgE (1:10000), concanavalin A (Con A. 10 ug per ml) and phorbol myristate acetate (PMA 10 ng per ml), and C5a (1:100). Basophils from ragweed-sensitive donors were preincubated with HRIF-containing supernatant (final dilution of 1:6) for five minutes, and then challenged with various agonists. Results (X±S.E.M) from six experiments are shown in the figure. HRIF significantly inhibited HRF-induced histamine release but failed to affect histamine release by allergen, anti-IgE, concanavalin A, PMA, and C5a.

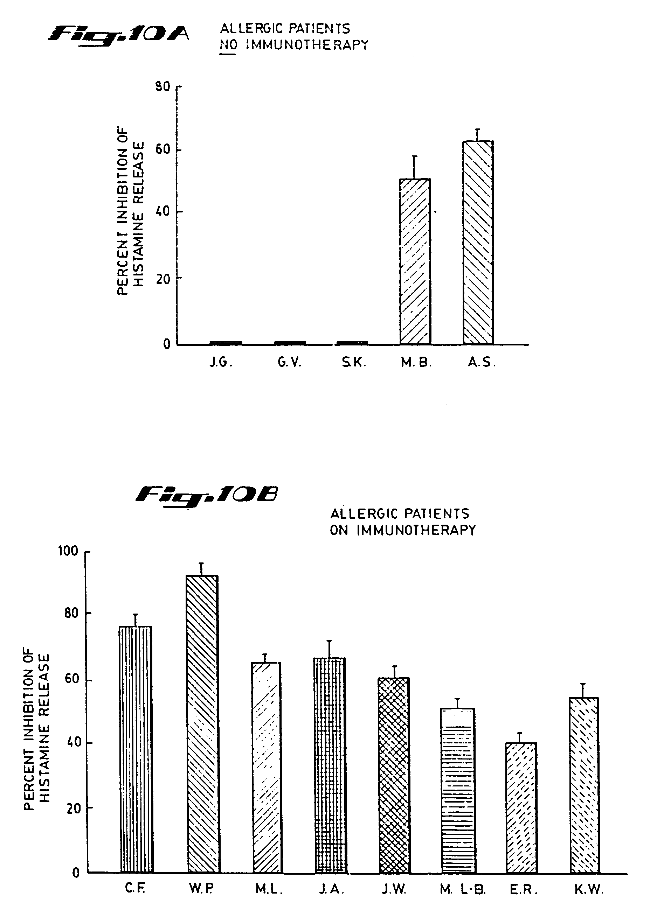

FIG. 10 -- Production of HRIF by MNC obtained from allergic patients without (10A) or with (10B) immunotherapy. Five symptomatic allergic patients who had never received immunotherapy and 8 allergic patients who were being treated with immunotherapy at maintenance doses were studied. MNC were isolated and cultured with histamine ($10^{-8}$ M) as described in FIG. 2. HRIF activity was determined at a dilution of 1:6 using HRF-induced histamine release. Control histamine release ranged from 20% to 44%. Cells from three out of five of the untreated group failed to release HRIF, whereas the treated group synthesized HRIF in the range of normal subjects (depicted in FIG. 2) For FIG. 10C, which shows a comparison of HRIF production from cells of healthy subjects, allergic patients, and allergic patients on immunotherapy experiments were performed as described in Example III.

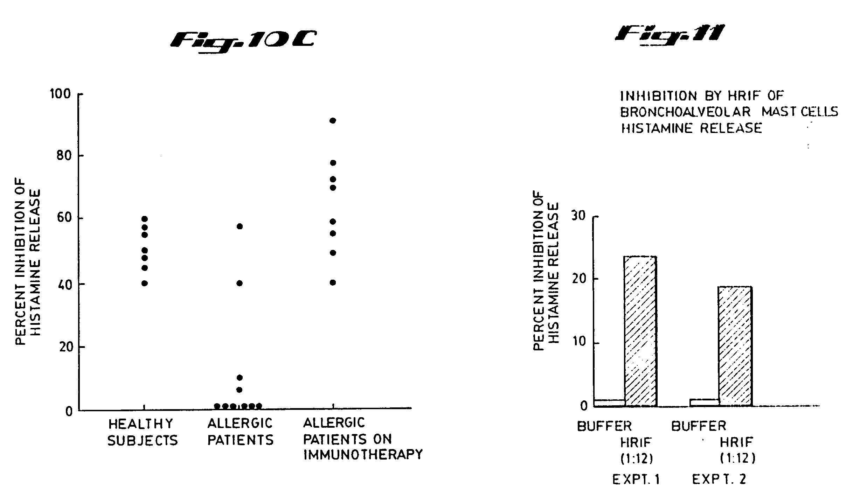

FIG. 11 -- HRIF-induced inhibition of histamine release from bronchoalveolar mast cells isolated from lavage fluid. Lavage cells were washed and preincubated with histamine-stimulated MNC supernatant and then challenged with HRF. Results are expressed as percent inhibition of histamine release. HRF-induced histamine release was 18% in experiment 1 and 38% in experiment 2.

FIG. 12 -- Unconcentrated Bronchoalveolar Lavage (BAL) samples were tested for the inhibition of HRF-induced histamine release using basophils from two allergic donors and one healthy subject. The control histamine release by HRF in the inhibition assay was in the range of 20-40%.

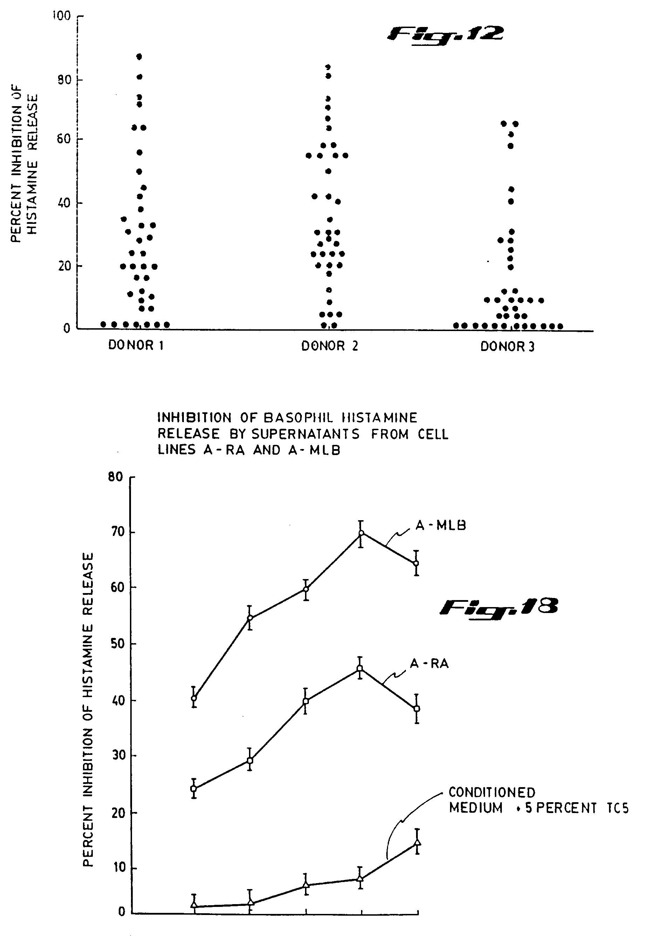

FIG. 13 -- Effect of dialysis on the inhibition of histamine release by BAL fluids. Samples of BAL fluids obtained from normal subjects and asthmatic patients were dialyzed overnight against buffer using Spectrapor dialysis membranes (MW cut-off 3,500) and then tested again for the inhibition of HRF-induced histamine release from basophils obtained from a healthy subject. The control histamine release was in the range of 25-40%.

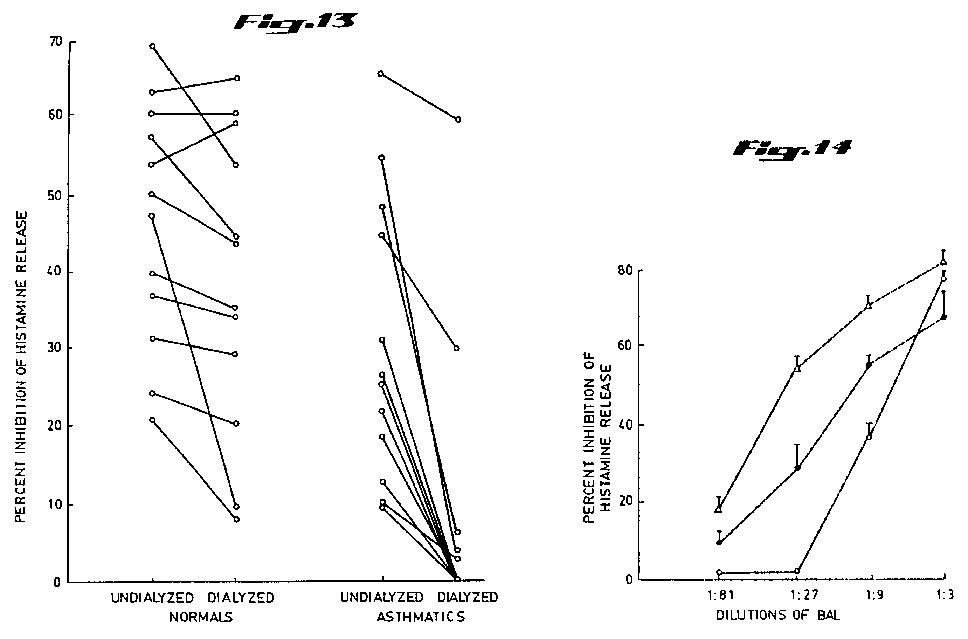

FIG. 14 -- Dose-dependent inhibition of HRF-induced histamine release by samples of dialyzed BAL fluids. Basophils from a healthy subject were preincubated with dilutions of three different dialyzed BAL samples and then challenged with HRF.

The control histamine release in this experiment was 25%.

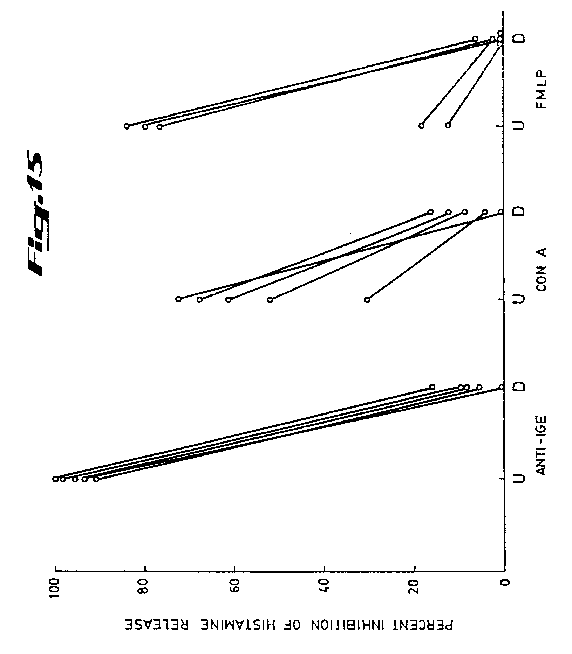

FIG. 15 -- Effect of undialyzed (U) and dialyzed (D) BAL fluids on the histamine release by FMLP, anti-IgE and Con A.

Basophils from a normal subject were preincubated with samples of BAL fluids and then challenged with anti-IgE (dilution $10^{-4}$), Con A (15 ug/ml) and FMLP ($10^{-5}$ M). Control histamine release by the secretagogoues was in the range of 20-50%.

FIG. 16 -- Gel-filtration profile of a concentrated BAL sample. BAL fluid from a normal subject was concentrated 33x and 3 ml sample was applied to a column containing Sephadex G-50. Fractions were screened for HRIF activity using basophils from a normal subject. Similar elution profile was seen in three separate experiments.

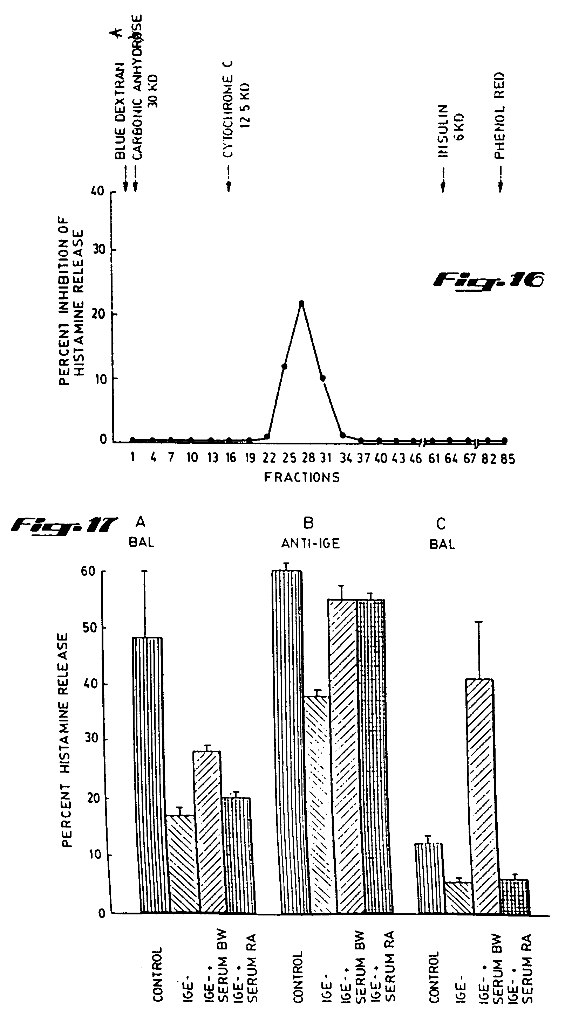

FIG. 17 -- Effect of lactic acid treatment of basophils on BAL-HRF-induced histamine release. Basophils isolated from an allergic asthmatic patient (Panel A and B) were treated with lactic acid for 3.5 min and then washed and resensitized with either serum from another allergic asthmatic patient (BW) or a normal subject (RA). The cells were then challenged with concentrated samples of BAL (Panel A) or anti-IgE (Panel B).

In another set of experiments, basophils from an atopic asymptomatic subject were treated similarly as described above and then resensitized with the allergic or normal serum (Panel C). The cells were subsequently challenged with a sample of concentrated BAL.

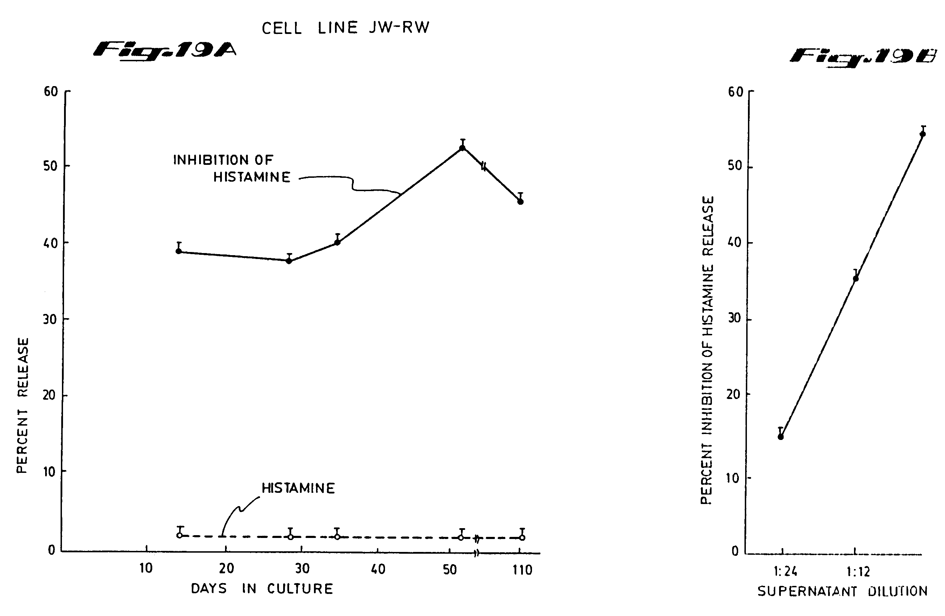

FIG. 18 -- Dose response of inhibition of basophil histamine release by supernatant from two cell lines (A-MLB and A-RA). Both cell lines originated from mononuclear cells stimulated with specific antigen (dust mite) and were maintained in defined medium (20% conditioned medium containing T-cell growth factor obtained from Cellular Products, Inc. and 10% fetal calf serum in RPMI). Dialyzed culture supernatant at various dilutions was tested for inhibition of HRF-induced histamine release from basophils as described earlier. Control histamine release was 20%. The modest inhibitory effect of conditioned medium and 10% fetal calf serum is also shown. Unfortunately, these two cell lines were lost due to contamination.

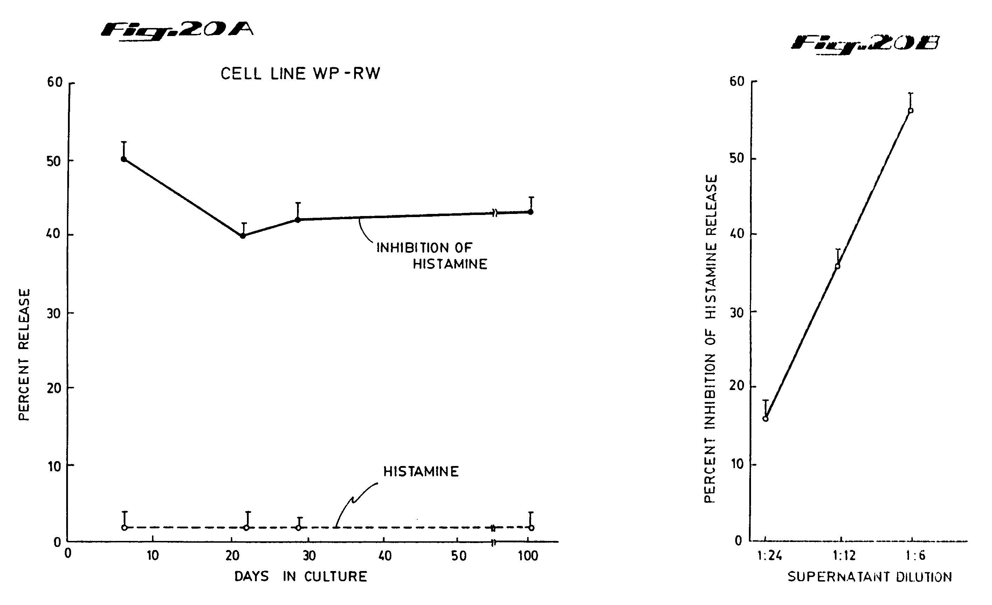

FIG. 19 -- Time course of production of HRIF (●----●) and HRF (o----o) by cell line JW-RW. Mononuclear cells from a patient on immunotherapy with ragweed pollen were isolated on Ficoll-Hypaque gradient, resuspended in RPMI-1640 plus glutamine, penicillin, streptomycin plus 15% heat inactivated fetal calf serum, and stimulated with ragweed antigen for two weeks at 37°C. in Corning 25 square cm tissue culture flasks. The cells were subsequently recultured in fresh medium containing 10 units per ml human recombinant interleukin 2 (rIL-2) and mitomycin C treated heterologous feeder cells. During the third and sixth weeks of culture, the cells were restimulated with ragweed pollen.

FIG. 20 -- Time course of production of HRIF (●----●) and HRF (o----o) by cell line WP-RW. Mononuclear cells from a patient on immunotherapy with ragweed pollen extracts were isolated as in FIG. 13 except that antigen-binding T cells were enriched by rosetting with AET-treated sheep red blood cells and panning on a ragweed-coated Petri dish.

## DETAILED DESCRIPTION OF A PREFERRED EMBODIMENT

The present invention describes the identification of a human cytokine designated as histamine release inhibitory factor (HRIF). As discussed in more detail below, this anti-allergic cytokine is a proteinaceous factor capable of inhibiting HRF-mediated release of allergic mediators from human- histamine containing proallergic cells. For the purposes of the present invention, a histamine containing proallergic cell may be defined as a cell of the immune system that contains histamine.

The investigations leading to the discovery of the present invention were directed toward determining whether leukocytes produce active substances that inhibit allergic reactions. In earlier experiments, several of the present inventors had discovered a factor(s) (termed "HRF") that causes the release of histamine and leukotrienes from basophils and mast cells. By the present disclosure, the inventors now report the discovery of a new anti-allergic cytokine (HRIF) capable of inhibiting the effects of HRF. Specific inhibition of HRF by HRIF is believed to represent an important physiologic mechanism for regulating release of allergic mediators from basophils and mast cells, and HRIF produced in vivo may represent a critical endogenous inhibitor of allergic sensitivity. Administration of exogenous HRIF is expected to prove useful in the treatment of asthma, other allergic diseases, and disorders where mast cells and basophils are thought involved with pathogenesis. HRIF may be particularly useful since it can be produced by human cells and may be better tolerated by humans than non-human drugs and substances. In addition, blood, serum and other biological fluid levels of this cytokine, or the synthesis of HRIF by blood cells in vitro, are likely to correlate with clinical status, and provide the basis for development of clinical assays (such as immunoassays) useful for monitoring patients before and after immunotherapy.

Therefore, in accordance with one aspect of the present invention, there is provided a substantially purified human histamine release inhibitory factor. This factor may be generally defined as an antagonist of HRF; that is, the factor is able to inhibit the effects of HRF; for example, HRF-induced histamine release.

In accordance with an additional aspect of the invention, there is provided a composition comprising a substantially purified proteinaceous factor exhibiting biologic properties that include inhibition of allergic mediator release from human mast cells or basophils triggered with HRF.

Of course, although the factor claimed in this embodiment is described as a proteinaceous factor, those of skill in the biological arts will understand that this terminology does not mandate that the factor consist entirely of protein.

Indeed, many biologically active substances comprise a protein chemically linked to another type of compound, for example, a carbohydrate or lipid moiety. Those of skill in the art should understand that the term "proteinaceous" is intended to include such compounds as glycoproteins, lipoproteins, and so forth.

Moreover, those of skill in the art will also appreciate that mast cells and basophils contain a number of enzymes and pharmacologic agents, including histamine and leukotrienes, that are generally referred to as allergic mediators. HRF has previously been shown to mediate release of at least two of these mediators, histamine and leukotrienes. (See, Ezeamuzie and Assem, Agents Actions, 13:222 (1983), incorporated herein by reference.) Although the precise biologic mechanism whereby HRIF exerts its activity is not completely understood, it is believed to counteract the effects induced by HRF. Therefore, while the present inventors have chosen a histamine release assay in order to demonstrate the anti-allergic activity of the compound of the present invention, it is likely that the compound inhibits release of other mediators, e.g. the leukotrienes, whose release is also induced by triggering the relevant cells with HRF. Of course, it should be pointed out that, as histamine is itself a potent allergic mediator, possession of an ability to inhibit HRF-induced histamine release is entirely sufficient to place a proteinaceous factor within the claims of the present invention.

Finally, the proteinaceous factor is characterized in that it inhibits allergic mediator release from human mast cells or basophils "triggered with" histamine releasing factor. Although the term "triggered with" is believed to be self-explanatory to those skilled in the art, a short definition may assist those not so skilled in understanding the invention. "Triggering" is a term of art used to describe treatment of basophils or mast cells so as to induce release of allergic mediators. Generally speaking, a cell is "triggered with" an agent when it is treated with that agent under conditions that normally induce mast cells or basophils to release allergic mediators such as histamine. This release may occur with an explosive degranulation or through non-cytotoxic secretion of certain mediators. All that is required is that the cells release an allergic mediator when "triggered with" (exposed to) the particular agent in question. It should also be appreciated that triggering can occur experimentally in the laboratory or clinically in vivo. Of course, the present invention provides a factor which inhibits allergic mediator release and it may seem paradoxical to some to say that cells are "triggered with" an agent when allergic mediator release is actually inhibited. For the purposes of the present invention, the term "triggered with" an agent is defined as meaning exposed to the agent in such a manner as to induce histamine or allergic mediator release under conditions where release is obtained in the absence of an effective amount of such an inhibitor.

The present inventors have shown the histamine release inhibitory factor of the present invention can be characterized as having a molecular weight of between about 8,000-10,000 daltons as determined by gel exclusion chromatography. Moreover, in some experiments, a second species having a molecular weight of about 20,000 to 30,000 daltons has been observed. Each of these species is considered to represent an embodiment within the scope of the present invention. Of course, it will be appreciated by those of skill in the art that gel filtration chromatography does not provide an exact molecular weight determination, rather, it provides a molecular weight based on the position at which the relevant inhibitory activity elutes from a sizing gel relative to the position at which markers of known molecular weight elute. Therefore, although this molecular weight determination is believed to be reasonably accurate, the molecular weight of the actual proteinaceous factor of the present invention may vary somewhat depending on experimental conditions used, for example, exclusion limit of the gel, column size, and the particular gel filtration methodology utilized. Of course, other methods for determining molecular weight, such as gel electrophoresis or high pressure liquid chromatography, exist. It will also be appreciated, therefore, that using differing measurement conditions, the molecular weight of HRIF may vary somewhat. The factor is further defined as being retained by a dialysis membrane having about a 3,500 molecular weight "cut-off." (This means that the dialysis membrane is generally impermeable to molecules having a molecular weight greater than about 3,500 daltons.)

It has also been observed that the factor may be substantially purified by ion exchange chromatography. For example, the present inventors have shown that unlike HRF, HRIF binds to DEAE cellulose (DE-52) (an ion exchange resin) equilibrated with 1 mM phosphate buffer, pH 7.4. The activity can be eluted from the gel with about 100 mM to 200 mM NaCl in the same buffer.

Various other characteristics of the factor have also been identified. For example, inhibition of allergic mediator release is significantly lowered when preparations containing the anti-allergic factor are treated with trypsin or chymotrypsin, enzymes known to cleave protein molecules at specific points. Therefore, one characteristic of the factor is lability of anti-allergic activity to chymotrypsin or trypsin treatment. The term "lability," as used herein, is defined as the loss of substantial biological activity following the indicated treatment as defined by the present disclosure, while the term "stability," as used herein, is defined as the retention of substantial biologic activity following the indicated treatment. The composition of the present invention may be further defined as exhibiting biologic stability following treatment with neuraminidase, or heat treatment at 60°C. for one hour, or freezing and thawing four times.

The factor has been compared with several other human biological factors commonly referred to as "cytokines." When directly compared with commercially-available preparations of recombinant interleukins 1, 2, and 4 and of gamma-interferon 3, tumor necrosis factor and GM colony stimulating factor, it was discovered that only histamine release inhibitory factor could block the release of histamine from basophils triggered by histamine releasing factor. The factor described in this invention is further distinguished from the aforementioned cytokines (interleukins 1, 2, and 4 and gamma-interferon) in that one species has a lower apparent molecular weight, namely 8,000 to 10,000 daltons as determined by gel exclusion chromatography.

It has been pointed out that the histamine release inhibitory factor of the present invention appears to be fairly specific. When basophils or mast cells are treated with a composition comprising this factor, then triggered with histamine releasing factor, histamine release is significantly inhibited. That is, the histamine release from the factor treated cells is significantly lower than the histamine release observed when the cells are treated with HRF alone. However, the present inventors have observed that when cells are treated with HRIF, then triggered with other agents known to induce release of allergic mediators, such as allergen, anti-IgE (where IgE is bound to Fc receptors on the cell surface), concanavalin A, phorbol myristate acetate, or C5a, no inhibition of histamine release is observed. Therefore, the proteinaceous factor of the present invention is generally defined as inhibiting histamine release otherwise induced by triggering cells with histamine releasing factor. More specific embodiments relate to the failure of the factor to inhibit histamine release induced by the other specific secretagogues tested.

However, as those of skill in the art will recognize, there are a number of agents known to mediate histamine release not tested by the present inventors, for example, compound 48/80, calcium ionophore A23187, eosinophil-derived major basic protein, neurotransmitters such as substance P, glycosylation enhancing factor, etc., and it is quite possible that the factor of the present invention may inhibit histamine release induced by those agents. Therefore, Applicants' discussion of the relative specificity of the anti-allergic factor should not be interpreted to mean absolute specificity since the factor of the present invention has not been tested with all possible agents that trigger histamine release. Certainly, a compound having the characteristics of the compound of the present invention, but shown to inhibit histamine release induced by an additional agent not tested by the present inventors, would fall within the scope of the present invention. Additionally, it should be pointed out that a number of cytokines have been shown to have multiple biological functions. Therefore, other biological activities of HRIF that may be identified subsequently will be encompassed by this invention.

The composition of the present invention may be prepared by culture or mononuclear cells under certain conditions or by development of a particular cell line which may be shown to produce the factor. The present inventors have prepared the factor in both ways.

In general, preparation from short term cultures of mononuclear cells appears to be the most feasible at the present time. This procedure generally comprises isolating leukocytes from an individual, obtaining a mononuclear cell fraction of the leukocyte preparation, and culturing the cells in a suitable medium to produce a conditioned supernatant. This conditioned supernatant may then itself be used as a source of HRIF or, it may be subsequently fractionated to produce a substantially purified preparation.

Due to their ready availability, peripheral blood leukocytes are preferred as a leukocyte source. However, as those of skill in the art will appreciate, various other leukocyte preparations may be used as well. For example, human splenocytes are also likely to provide an acceptable source of the leukocytes, as are human mononuclear cells derived from thoracic duct drainage, lymph-node biopsy, tonsillar lymph-node biopsy, and so forth. In general, particularly when peripheral blood is used as a source of leukocytes, it is desirable to fractionate the peripheral blood prior to culture to obtain a mononuclear cell fraction, defined herein as a cell suspension comprising predominantly mononuclear cells. This is most readily accomplished by centrifuging the cells through a gradient of Ficoll-Hypaque according to the procedure described by Boyum in Scand. J. Clin. Lab. Invest., 21:suppl. no. 97, p. 77 (1968).

Of course, other methods known to those of skill in the art, for example, flow cytometric cell sorting, counterflow centrifugal elutriation, positive or negative selection using monoclonal antibodies to cell surface markers, or Percoll gradient centrifugation may also be used to prepare a mononuclear cell fraction. In addition, some sources of human leukocytes, such as lymph nodes, may inherently contain a predominantly mononuclear cell fraction so that procedures like those described above are not required. For example, lymph node cells may be teased from the lymph node, washed in a suitable buffer, and directly introduced into culture, while splenocytes may simply be treated to substantially remove red blood cells, washed in a suitable buffer, and then directly introduced into culture. However, where peripheral blood cells are used as a source of the leukocytes, it is preferred that the mononuclear cell fraction be prepared by Ficoll-Hypaque centrifugation as previously described.

Once a mononuclear cell fraction is obtained, it is then cultured in a suitable medium to produce a conditioned supernatant. A preferred medium is RPMI-1640 supplemented with 10 mM Hepes buffer, 4 mM glutamine, $5 \times 10^{-6}$ M 2-mercaptoethanol, and various antibiotics, such as penicillin and streptomycin. Of course, those of skill in the art will recognize that a number of tissue culture mediums suitable for culture of human lymphoid cells exist (for example, Eagle's minimal essential medium, Dulbecco's modified medium, or Iscove's modified medium, etc.) and substitution of one tissue culture medium for another is considered to be well within the skill of the art. In general, best results are obtained when the cells are cultured for a period of 24 hours at 37°C. in a humidified incubator containing 5% $CO_2$. The present inventors have found that the optimal density of cells introduced into the culture to be $3 \times 10^6$ per ml. Of course, those of skill in the art may find that certain culture conditions may be modified somewhat without significantly affecting factor production. For example, it may be possible to culture cells at a higher density for a shorter period of time, or conversely, to culture cells at a lower density for a longer period of time. Such modifications are considered to be well within the scope of the present invention.

The present inventors have clearly discovered, however, that addition of histamine to the culture medium significantly increases the allergic mediator release inhibitory activity of the conditioned supernatant. For this reason, it is especially preferred that histamine, preferably at a concentration of between about $10^{-6}$ to $10^{-10}$ M, be incorporated into the culture medium. Although the precise mechanism whereby histamine increases HRIF production is not completely understood, it is possible that histamine may preferentially induce the synthesis of HRIF, while decreasing the synthesis of HRF. The synthesis of HRIF is also increased when MNC are cultured in the presence of mitogens (specifically concanavalin A) or allergens (for example, dust mite or ragweed pollen allergenic extracts). In the latter case, the cells cultured with allergens should come from allergic donors. It is anticipated that synthesis of HRIF would also be obtained by culturing cells with other mitogens and allergens.

After the cells have been cultured for the requisite period of time, a conditioned supernatant is then prepared from the cell culture. The conditioned supernatant is quite simple to prepare and may be prepared in a number of ways. An extremely simple method for preparing the conditioned supernatant is allowing the cultured cells to settle to the bottom of a culture vessel and then removing the liquid remaining above the cells. Alternatively, the cell suspension may be centrifuged, the cells pelleted, and the conditioned supernatant decanted.

Although conditioned supernatant produced as described above has been consistently shown to contain HRIF activity, in some cases, it may be desirable to fractionate the supernatant to obtain a fraction which includes the factor in a more purified form. Of course, where the supernatant is derived from a cell culture containing added histamine, it is important to substantially reduce the concentration of histamine in the supernatant so that it will not interfere with the histamine release assay used to detect histamine release inhibitory factor. Therefore, it will often be desirable to use a procedure to substantially remove histamine from the culture supernatants, particularly where such culture supernatant is not fractionated by procedures which would themselves separate the anti-allergic factor from histamine. This is most simply achieved by dialyzing the culture supernatant as described in Example I below.

The supernatant may then be fractionated to produce a substantially purified preparation of HRIF. A preferred method for fractionating the supernatant comprises fractionation on the basis of molecular weight. A number of techniques for fractionating proteins on the basis of molecular weight are known to those of skill in the art and may, in some cases, be used. However, in many cases, it will be desirable that the protein fraction isolated retain biological activity. Therefore, as a general matter, it is preferred that the fractionation procedure not require denaturation of the proteinaceous factor. For this reason, gel exclusion chromatography is a generally preferred purification method. Of course, those of skill in the art will recognize that a number of suitable gels may be used to purify proteinaceous molecules. The present inventors have discovered that chromatography over a Sephadex G-50 superfine gel is an acceptable procedure for fractionating the supernatant to provide a HRIF enriched fraction.

Alternatively, the supernatant may be fractionated by ion exchange chromatography. Under the experimental conditions used (1 mM phosphate buffer, pH 7.4), HRIF was bound to DEAE (DE-52) cellulose and was eluted from the resin with about 100 mM to 200 mM NaCl in the same buffer. Of course, it will be recognized that, with hindsight provided by the present inventors' discovery, other resins, binding buffers, and elution buffers could also be used to fractionate a conditioned supernatant containing HRIF so as to produce a fraction containing HRIF that is substantially purified away from the other factors likely to be present in the supernatant. For example, the inventors have shown that HPLC ion exchange chromatography is also suitable for purifying the factor.

The selected cell supernatant or fractions thereof are then tested for histamine release inhibitory activity. In general, this procedure utilizes cell preparations enriched for basophils or mast cells from human donors. These cells may be isolated according to known procedures. For example, where basophils are used as the test cells, cell preparations enriched for basophils may be prepared from peripheral blood by sedimentation with hydroxyethyl starch according to the procedure described by Thueson, et al., J. Immunol., 123:626 (1979). Mast cells may be obtained by bronchoalveolar lavage of subjects with bronchial asthma essentially as described by Bernstein, et al., in J. Allergy Clin. Immunol., 76:145 (1985). The present inventors have also observed that HRIF can be recovered in bronchoalveolar lavage fluid and is more readily detected in fluids from normal subjects than in those with bronchial asthma. This finding supports the physiologic role of HRIF in preventing development of allergic disorders such as bronchial asthma.

The actual assay for the histamine release inhibitory factor is described below. In general, cells are first exposed to an aliquot of either a control buffer or an aliquot of the supernatant or supernatant fraction to be tested. The cells are then triggered with HRF under standardized experimental conditions which have previously been shown suitable for measuring HRF induced histamine release. For example, see Thueson, et al., J. Immunol., 123:626 and 633 (1979). The amount of histamine released by the two sets of samples is then determined by known procedures (see Siraganian, J. Immunol. Methods, 7:283 (1975) and Verburg, et al., Life Sciences, 32:2855 (1983)) and inhibition of histamine release is calculated as described in Example I.

Of course, as indicated above, the isolation of HRF from a number of sources has been previously described. While it may be likely that these other sources of HRF may successfully be used in the above assay with possible routine modifications of experimental conditions, the present inventors have successfully used HRF prepared as described in Example I. Accordingly, this preparation of HRF is preferred for use in the assay.

In addition to describing isolation of HRIF from human mononuclear cell cultures, the present inventors have also produced several human cell lines that produce the factor. Several procedures have been used to generate these cell lines. For example, in one case, two HRIF producing cell lines were produced by stimulating mononuclear cells from allergic patients on immunotherapy with specific allergens and maintaining the cells in defined medium containing T-cell growth factor as well as mitomycin-treated heterologous feeder cells. In a more preferred protocol, the mononuclear cells are isolated from patients on immunotheraphy and restimulated in vitro with at least one antigenic component of the immunotherapeutic suspension used to treat the particular patient. This in vitro stimulation is continued for approximately two weeks. Thereafter, the cells may be maintained by culturing with human recombinant interleukin-2 and mitomycin-treated feeder cells. In a preferred procedure, the cells are restimulated on two more occasions by antigen. In a third procedure, antigen-binding T cells are isolated from mononuclear cells by rosetting with AET-treated sheep red blood cells and panning the cells with allergen-coated Petri dishes, essentially as described by Pellegrino, et al., Clin. Immunol. Immunopath., 3:324 (1975) and Taniguchi and Miller, J. Exp. Med., 146:1450 (1977). Subsequently the cells are stimulated periodically with specific allergen and maintained with interleukin-2 and feeder cells. These procedures are described in more detail in Example V.

Since the anti-allergic factor of the present invention is likely to be used primarily as a therapeutic agent for treatment of allergic disease, the invention also encompasses methods for inhibiting release of allergic mediators from human basophils or human mast cells by exposing the cells to a composition comprising an effective amount of the factor. This amount may be empirically determined in the laboratory by procedures similar to those described in Example I by clinical trial, and by other means known to those of skill in the art.

Of course, the composition of the present invention may also be formulated with a suitable pharmaceutical excipient or carrier to prepare an anti-allergic preparation. Suitable carriers may include, but are not limited to, human serum albumin and mannitol, and other carriers well-known to those of skill in the pharmaceutical arts. The composition may also contain preservatives such as phenol and thimerosal. Finally, the invention encompasses a method for treating an individual comprising administering to that individual a therapeutically acceptable dose of an anti-allergic preparation containing substantially purified HRIF. In general, empirical procedures will be required in order to determine the optimal dose for treating a particular allergic condition. These procedures are generally known to those of skill in the pharmaceutical arts and will be discussed in some additional detail in Example VI.

In addition, in the future, the HRIF might be prepared by recombinant DNA technology. The cloning of the gene for HRIF might be done in several ways. One of the preferred methods will be to isolate RNA from histamine-stimulated MNC. mRNA could be isolated from the RNA by using oligo (dT)-cellulose and translated in frog oocytes. Once the mRNA for HRIF is detected, a cDNA library could be constructed and translated in bacteria or yeast using an appropriate vector (e.g. pUC9). The specificity of the cDNA could be tested by hybridizing with mRNA from MNC. Once the specificity is determined, the cDNA will be sequenced. Although this method is preferred, other methods, for example, synthesis of an oligonucleotide from a partial amino acid sequence, might also be used.

The following examples are provided in order to more clearly illustrate the invention.

EXAMPLE I

METHODS FOR PRODUCING HRIF

GENERATION OF HRIF BY NORMAL HUMAN MONONUCLEAR CELLS

The following example is designed to illustrate the production of the anti-allergic cytokine by normal human mononuclear cells.

A. Reagents

RPMI-1640 was obtained from GIBCO Laboratories, Grand Island, NY; fetal calf serum from HyClone Laboratories, Inc., Logan, UT; glutamine, 2-mercaptoethanol, histamine, Ficoll, Hypaque, human serum albumin (HSA), phorbol myristate acetate, concanavalin A (Con A), penicillin, streptomycin and agarose-coupled trypsin, chymotrypsin and neuraminidase from Sigma Chemicals Co., St. Louis, MO; Hepes from Research Organics, Inc., Cleveland, OH; tissue culture flasks from Corning Glass Works, Corning, NY; Sephadex G-50 superfine from Pharmacia, Inc., Piscataway, NJ; hydroxyethyl starch (HetaStarch) from American McGaw, Irvine, CA; Spectrapor dialysis tubing from Spectrum Medical Industries, Los Angeles, CA; human recombinant interleukin 1, 2, and 4 from Genzyme, Boston, MA; and human recombinant gamma-interferon from Collaborative Research, Inc., Bedford, MA; DEAE (DE-52) gel from Whatman, Ltd., England; ragweed pollen allergenic extract from Greer Laboratories, Lenoir, NC; house dust mite allergenic extract from Hollister-Stier Laboratories, Spokane, WA; rabbit anti-human IgE serum from Behring Diagnostics, Somerville, NY. C5a was prepared as described by Farnam, et al., J. Immunol., 134:541 (1985).

## B. Isolation Of Mononuclear Cells (MNC) From Peripheral Blood

Peripheral venous blood was drawn from healthy donors after obtaining written informed consent. Heparinized blood was diluted with two volumes of Hanks balanced salt solution (HBSS) and centrifuged on a Ficoll-Hypaque (specific gravity 1.077) cushion. Mononuclear cells were separated and washed three times with HBSS and suspended at a concentration 5 X 10$^6$ per ml in RPMI-1640 medium containing 100 units penicillin per ml, 100 mcg streptomycin per ml, 4 mM glutamine, and 5 X 10$^{-6}$ M 2-mercaptoethanol.

## C. Isolation Of Basophil Enriched Peripheral Blood Leukocytes

Basophil enriched leukocytes were isolated from peripheral blood of normal or allergic donors according to the following procedure. Venous blood from donors was anticoagulated with 10 mM EDTA and sedimented with 1.5% hydroxyethyl starch for 30 minutes at room temperature. The leukocyte-rich buffy coat was collected and washed three times in HA buffer (10 mM Hepes, pH 7.4, plus 137 mM NaCl, 5 mM KCl and 0.03% human serum albumin) and the cells pelleted in a refrigerated centrifuge (4°C.) at 300 X G for 10 minutes. The washed leukocytes were suspended in HACM buffer (Hepes buffer, pH 7.4, 0.03% human serum albumin, 2 mM CaCl$_2$ and 1 mM MgCl$_2$). The preparation contained about 0.5% basophils. Routinely it is not necessary to purify basophils further since these cells contain virtually all the blood histamine. Leukocytes from 1 ml of blood were usually used for one duplicate experiment.

## D. Generation Of HRF-Containing Supernatant And Assay Of HRF

MNC were pulsed with concanavalin A (25 ug per ml) in RPMI medium for four hours, washed twice with HBSS, resuspended in RPMI medium, and then cultured for 72 hours. Supernatants were harvested and concentrated 50-fold using an Amicon ultrafiltration chamber with YM-5 filters (MW cut-off 5,000). Usually supernatants were pooled from cultured mononuclear cells obtained from 10-15 liters of blood and then applied to gel filtration columns (Sephadex G-75). Fractions containing histamine releasing activity (15K-30K fractions) as measured by leukocyte histamine release test were pooled, aliquoted, frozen at -70°C. and used as a source of HRF.

For assay, aliquots of 100 ul of test supernatant were incubated with 100 ul of leukocyte suspension for 45 minutes in a shaking water bath at 37°C. Each experiment was done in duplicate. Four hundred microliters of HA buffer was added to each tube at the end of the incubation. The supernatants were separated from the cells by centrifugation at 600 X g for five minutes at 4°C. The histamine content of the supernatants was measured using an automated fluorometric analyzer (Siraganian, J. Immunol., methods, 7:283 (1975)). Spontaneous histamine release was assessed by incubating the cells in HACM buffer alone. The total histamine content of the cells was measured by lysing the cells with 3% perchloric acid. The percentage of histamine release was calculated according to the formula:

$$\frac{\text{histamine in the supernatant}}{\text{total histamine in the cells}} \times 100$$

Spontaneous histamine release from the cells was usually less than 5% and was subtracted from the calculated histamine release.

In a modification of this protocol, mononuclear cells from 15 healthy donors were cultured alone or in the presence of histamine (10$^{-6}$ M) for 24 hours. Supernatants were dialyzed and tested for histamine releasing activity, essentially as above. The results of this experiment, shown in FIG. 1, indicated that a significantly lower amount of histamine release was obtained when histamine was added to the cultures. This finding could result from histamine-mediated suppression of HRF production, histamine-mediated stimulation of synthesis of an inhibitor, or a combination of the two.

## E. Generation Of HRIF Containing Supernatant And Assay Of HRIF

Mononuclear cells (3 X 10$^6$ cells per ml) were cultured alone in RPMI-1640 (with added glutamine 4 mM, 2-mercaptoethanol 5 x 10$^{-6}$M, penicillin 100 units per ml, and streptomycin 100 mcg per ml) or in the presence of varying concentrations of histamine in the same medium for 24 hours. In other experiments, mononuclear cells were stimulated with concanavalin A and with pollen allergens. Culture supernatant was harvested by centrifugation at approximately 450 X g for ten minutes at 48°C., dialyzed in Spectrapor-3 tubing (molecular weight cutoff = 3,500 daltons) twice against 1000 volumes of distilled water at 4°C. for 12 hours and 100 volumes of 10 mM Hepes buffer, pH 7.4, (containing 137 mM NaCl, 5 mM KCl, 2 mM CaCl$_2$, and 1 mM MgCl$_2$) for 4 hours in order to remove histamine. The dialyzed supernatants were used immediately or stored at -70°C. This supernatant was tested for inhibition of HRF-induced histamine release from basophils isolated according to the procedure described in Example I C.

Aliquots of 100 ul of diluted test supernatants were preincubated with 100 ul of leukocyte suspension (2-5 X 10$^6$ cells per ml) for five minutes at 37°C. and then challenged with 100 ul of HRF-containing supernatant for an additional 45 minutes at 37°C. Three sets of controls were run simultaneously: 1) cells preincubated with buffer and then challenged with HRF; 2) cells preincubated with the test supernatant and then challenged with buffer; and 3) cells preincubated with buffer and challenged with buffer. Each experiment was done in duplicate. At the conclusion of the incubation, 300 ul of HACM buffer was added to each tube and the supernatants were separated from the cells by centrifugation at 600 X g for five minutes at 4°C. The histamine content of the supernatants was measured spectrofluorometrically. The percentage of histamine released was calculated as described above.

A typical experiment was carried out according to the following protocol:

| Preincubation | Challenge |
| --- | --- |
| a. leukocytes + buffer | + buffer |
| b. leukocytes + buffer | + HRF |
| c. leukocytes + supernatant | + buffer |
| d. leukocytes + supernatant | + HRF |

The percentage of inhibition of histamine release was calculated according to the formula:

$$[(b\text{-}a)\text{-}(d\text{-}c)] \times 100/(b\text{-}a)$$

As stated previously, early experiments showed that culture of mononuclear cells in the presence of histamine resulted in decreased histamine release. This finding could result from histamine-mediated suppression of HRF production, histamine-mediated stimulation of synthesis of an inhibitor, or a combination of the two.

Therefore, in order to determine whether an inhibitor was produced, the supernatant fluid from normal human MNC cultured with histamine at $10^{-8}$ M was assayed for its ability to inhibit the release of mediators from peripheral blood basophils triggered with HRF. As shown in FIG. 2, histamine-stimulated MNC supernatant inhibited histamine release from basophils triggered with HRF. The action of this histamine release inhibitory factor (HRIF) obtained from six consecutive cell donors was dose-dependent over a wide range of dilutions. Conditioned supernatants produced by MNC cultured without histamine showed modest inhibitory activity. Histamine incubated with medium alone and then dialyzed did not show any inhibitory activity. Histamine was undetectable, less than $10^{-9}$M, (as determined by a sensitive radioenzymatic assay of Verburg, et al., Life Sciences, 32:2855 (1983)) in all these dialyzed supernatants.

When the recovery of HRIF from histamine-stimulated MNC was examined, significant activity was obtained with MNC from ten additional normal donors.

The following experiment was then performed in order to determine whether the enhanced HRIF production observed upon culture of mononuclear cells in the presence of histamine was dependent on the concentration of histamine in the culture medium and to establish the optimal histamine concentration for achieving enhanced HRIF production. MNC were harvested and cultured as described above except that the concentration of histamine added to the culture medium varied from $10^{-12}$ M to $10^{-6}$ M. Then, dialyzed supernatants were tested for inhibition of HRF-induced histamine release from basophils as described above. The results of this experiment, shown in FIG. 3, indicate that HRIF production was dependent on the amount of histamine present within the culture. Optimal HRIF production was achieved when the concentration of histamine in the culture medium was about $10^{-6}$ to $10^{-10}$ M. In these experiments, control HRF-induced histamine release ranged from 33%-40%. Although histamine has been reported to inhibit antigen-induced histamine release from basophils at concentrations of $3 \times 10^{-7}$ to $10^{-6}$ M, it is extremely unlikely that inhibition of HRF-induced histamine release was due to the presence of contaminating histamine in the supernatant since histamine was undetectable in the dialyzed supernatants (less than $10^{-9}$ M). Furthermore, HRIF does not appear to inhibit antigen-induced histamine release.

In addition, the following experiments were performed in order to further characterize HRIF mediated inhibition of allergic mediator release. Basophil enriched leukocytes were preincubated at room temperature with HRIF for 40-60 seconds, 3, 5, or 10 minutes and then challenged with HRF. The results of this procedure, shown in FIG. 4, indicated that a preincubation period of 5 to 10 minutes with basophils is necessary for complete inhibitory activity. Preincubation of basophils with HRIF for less than 5 minutes resulted in less inhibition of histamine release. Moreover, in additional experiments (FIG. 5), it was shown that removal of HRIF following the incubation period by repeated washes did not affect inhibition of histamine release from basophils. When HRF and HRIF were preincubated prior to addition of basophils, there was no change in the release of histamine suggesting that the two factors do not bind together in the fluid phase. Results are representative of three similar experiments.

Stimulation of HRIF production by histamine suggests that a negative feedback mechanism may exist between lymphocytes and histamine producing cells that would function as follows: lymphocytes produce HRF that activates mast cells and basophils to release histamine; this histamine then stimulates lymphocytes to produce HRIF which, in turn, inhibits further histamine release, thereby reestablishing immunologic homeostasis. Thus, there appears to be a fine balance between the production of HRF and HRIF in vivo and loss of this balance may contribute to the development of allergic disease. This hypothesis is further supported by the results of a study described in Example III below.

EXAMPLE II

CHARACTERIZATION OF HRIF

The following example describes the characterization and substantial purification of HRIF.

A. Physicochemical Characterization Of HRIF

Conditioned supernatant from histamine stimulated mononuclear cells was prepared as described above in Example I and used as a source of HRIF. Histamine-stimulated mononuclear cell supernatant was then tested for inhibition of HRF-induced histamine release after the following treatments: a) heating at 60°C. for one hour; and b) repeated freezing and thawing (4X). The results of these experiments, shown in Table I, indicated that HRIF is resistant to heat treatment at 60°C. for one hour and is relatively resistant to repeated freezing and thawing.

In addition, HRIF was subjected to digestion with various enzymes. For this procedure, trypsin and chymotrypsin coupled to agarose were washed twice with HCM, pH 7.8, and suspended in the same buffer. Aliquots of HRIF-containing supernatants from histamine-stimulated MNC cultures were incubated with trypsin (2 units per ml) or chymotrypsin (2 units per ml) for two hours at 30°C. Neuraminidase coupled to agarose was washed with acetate buffer, pH 5.0, and then incubated for two hours at 37°C. at a concentration of 2 units per ml with HRIF dialyzed against the same buffer. Controls included supernatants treated with the same buffers (positive control) and agarose-coupled enzymes incubated with buffer (negative control). Supernatants were separated from the enzymes by centrifugation, dialyzed against HACM buffer, and assayed for HRIF.

Enzymatic treatment of HRIF-containing supernatants with trypsin and chymotrypsin almost completely abolished its activity (Table I). This suggested that intact protein structure is necessary for the inhibitory activity of HRIF. Treatment of HRIF-containing supernatant with neuraminidase did not significantly affect its activity, suggesting that HRIF does not have sialic acid residues necessary for function.

Table I

| PHYSICOCHEMICAL CHARACTERIZATION OF HRIF | | | |
|---|---|---|---|
| Treatment | % inhibition of histamine release X ± SEM | | |
| | expt 1 | expt 2 | expt 3 |
| Untreated HRIF[a] | 82±2 | 38±2 | 64±2 |
| HRIF, 60°C. 1 hr | 84±4 | 35±1 | 60±2 |
| HRIF 4x freezing and thawing | 74±5 | 19±2 | 52±4 |
| HRIF + trypsin[b] | 36±5 | 0 | 8±2 |
| HRIF + chymotrypsin[b] | 0 | 0 | 0 |
| HRIF + neuraminidase[c] | ND | 30±6 | 54±4 |
| ND: Not done. | | | |

a: Untreated controls of HRIF for enzyme-treated experiments were dialyzed against HCM, pH 7.8, and then incubated at 30°C. for trypsin and chymotrypsin experiments or dialyzed against acetate buffer, pH 5, and then incubated at 37°C. for neuraminidase experiments for two hours. These treatments did not affect the activity of the supernatants.
b: HRIF was dialyzed against HCM, pH 7.8, and then incubated at 30°C. for two hours.
c: HRIF was dialyzed against acetate buffer, pH 5.0, and then incubated with neuraminidase in the same buffer at 37°C. for two hours.

Finally, experiments were performed in order to differentiate HRIF from several known cytokines. For these experiments, basophils were preincubated with various concentrations of human recombinant interleukin 1 (IL-1) beta, IL-2, IL-4, and gamma-interferon, and then challenged with HRF. None of these cytokines at a concentration up to 500 units per ml inhibited HRF-induced histamine release (FIG. 6A). Similarly, HRIF has been shown to be distinct from tumor necrosis factor, IL-3, & G.M. colony stimulating factor (FIG. 6B).

B. Purification Of HRIF Activity

HRIF was purified by gel exclusion chromatography of histamine-stimulated MNC supernatant. Generally, about 2 ml of concentrated supernatant (10-15 X) was applied to a column (90 cm X 1.5 cm) packed with Sephadex G-50 and equilibrated with HCM buffer. The column was calibrated with blue dextran, carbonic anhydrase, cytochrome C, and insulin. Flow rate was maintained at 12 ml per hour at 4°C. One milliliter fractions were collected and tested for inhibition of HRF-induced histamine release from basophils as outlined in Example I. The results of this experiment, shown in FIG. 7A, demonstrate that HRIF activity eluted in a region consistent with a molecular weight of 8,000-10,000. In this experiment, control histamine release was 26%. The experiment was repeated with two different lots of HRIF and a similar elution profile of HRIF was noted each time.

In order to determine whether concanavalin A-stimulated supernatant contained both HRF and HRIF activities, gel chromatography of concanavalin A-pulsed MNC supernatant was performed and fractions were assayed separately for HRF and HRIF activities. As shown in FIG. 7B, HRF was recovered from fractions which corresponded to the MW ranges 15K-30K as well in a larger MW fraction (previously shown to be approximately 50K), and HRIF appeared in the MW range 8K-10K. Histamine-stimulated supernatant from MNC of some normal donors also showed a small peak of HRF in the MW range 15K-30K (see FIG. 7A).

However, a slightly different profile was observed in other experiments (7C). In these experiments, twenty-fold concentrated supernatant (2 ml) was applied to a column (90 cm x 1.5 cm) that was precalibrated with marker proteins. One milliliter fractions were collected, and every other fraction was tested for direct histamine release (o) and for the inhibition of HRF-induced histamine release (●) from basophils. This profile of HRIF activity, which shows an additional peak of activity at about 20,000 to 30,000 daltons, has been observed in 1 of 3 MNC donors. It is possible that the higher molecular weight species represents a multimer of the lower molecular weight form.

HRIF was also purified by ion exchange chromatography. For this procedure, concanavalin A supernatant was dialyzed against 1 mM phosphate buffer, pH 7.4, and incubated overnight with DEAE cellulose that was then put in a small column, washed with the same buffer, and then eluted with a linear gradient from 1 to 500 mM NaCl in 1 mM phosphate buffer, pH 7.4, and the fractions collected. As shown in FIG. 8, HRIF was bound to the gel under the starting conditions and most of the activity eluted at approximately 100 mM NaCl. This experiment was repeated with a histamine-stimulated MNC supernatant; HRIF also bound to DEAE cellulose under similar conditions and could be eluted with 100 mM NaCl.

In a variation on this procedure, DEAE-cellulose purified HRIF in 1 mM phosphate buffer, pH 6.9 was applied to a Synchropak AX 300 anion exchange HPLC column that was equilibrated with the same phosphate buffer. After washing the column with the buffer for 20 min, a linear gradient of 0-0.5M NaCl was applied for 60 min and then further eluted with 0.5M NaCl in phosphate buffer for another 20 min. One milliliter fractions were collected, dialyzed and then assayed for HRIF activity using basophils from a healthy subject. Results of this procedure are shown in Figure 8A.

In another purification using HPLC gel filtration, two hundred fifty microliters of fifty-fold concentrated Con A-stimulated MNC supernatant was applied to a TSK 2000 gel filtration SW column preequilibrated with HCM buffer, pH 7.3. The column was eluted with the buffer at 0.3 ml/min and 0.6 ml fractions were collected and tested for HRIF activity. For this purpose, leukocytes from normal subjects were incubated with each fraction for 5 min and then challenged with a batch of HRF. The control histamine release was 55%. The optical density was monitored at 280 nm. Similar result was obtained with 5 different MNC supernatants. The results of this experiment indicated that the activity eluted with an apparent molecular weight of 15,000 to 25,000 daltons. The variation observed between the results shown in this figure and in FIG. 7A and 7B may be attributed to variations in the particular experimental conditions used.

In additional experiments, the inventors attempted to separate HRF and HRIF by affinity chromatography using concanavalin A-Sepharose, lentil lectin-Sepharose, and peanut agglutinin-Agarose. Neither molecule bound to these lectin-coupled gels (data not shown), suggesting that both HRF and HRIF, perhaps, do not contain mannose-, glucose-, or galactose-rich carbohydrates.

C. Specificity Of The HRIF Induced Inhibition

A number of compounds are known to stimulate release of allergic mediators from mast cells and basophils. The following example is designed to demonstrate the specificity of the inhibitory activity of HRF. In this example HRIF is shown to inhibit histamine release induced by triggering basophils with HRF, but not to inhibit histamine release induced by triggering basophils with any of the other compounds tested; namely, ragweed antigen, concanavalin A, anti-IgE, phorbol myristate acetate, and C5a (FIG. 9). Basophils from ragweed-allergic donors were preincubated with HRIF containing supernatant produced as described in Example I for five minutes, and then challenged with various secretagogues essentially as described in Example I. Cells were challenged with HRF, ragweed allergen ($10^{-6}$ w/v), anti-IgE (1:10,000 dilution of the antiserum), concanavalin A (10 ug per ml), phorbol myristate acetate (10 ng per ml), and C5a (1:100 dilution of partially purified anaphylatoxin prepared according to the Farnam, et al., J. Immunol., 134:541 (1985).

EXAMPLE III

SYNTHESIS OF HRIF BY MONONUCLEAR CELLS OF ALLERGIC SUBJECTS

The following example demonstrates that HRIF is made in deficient amounts by mononuclear cells from some allergic patients. Cells from another group of allergic patients receiving conventional allergen immunotherapy at maintenance doses, showed normal levels of synthesis.

Mononuclear cells from allergic donors were isolated and cultured with histamine ($10^{-8}$ M) for 24 hours essentially as described in Example I and in FIG. 2. Following extensive dialysis, HRIF was assayed using inhibition of an HRF standard which gave a control release of histamine or 20%-44%. For three out of five subjects not receiving immunotherapy, no significant synthesis by mononuclear cells was detected (FIG. 10A). Eight other subjects who were receiving maintenance doses of effective allergen immunotherapy were also studied (FIG. 10B). The level of HRIF recovered from supernatant fluid of their cells stimulated with histamine was not different from values obtained for nonallergic volunteers that are reported in FIG. 2.

In additional studies, MNC from 7 healthy subjects, 10 patients with allergic rhinitis/asthma and 8 allergic/asthmatic patients receiving immunotherapy were isolated and cultured with histamine ($10^{-8}$ M) for 24 hr. The culture supernatants were dialyzed to remove histamine and then tested for the inhibition of HRF-induced histamine release from basophils. For this purpose, leukocytes were isolated from an asymptomatic atopic (skin test-positive) subject, preincubated with MNC-supernatant and then challenged with a stock batch of HRF. The control histamine release in this experiment was 50%. These results are shown in Fig. 10C.

EXAMPLE IV

A. Demonstration of HRIF Activity on Human Mast Cells

The following example demonstrates that HRIF inhibits allergic mediator release from human mast cells.

Mast cells were obtained from human bronchoalveoli by bronchoalveolar lavage performed according to the procedure described in Bernstein, et al., J. Allergy Clin. Immunol., 76:145 (1985). Asthmatic subjects who were relatively stable were lightly sedated and the bronchoscope was passed to a segmental bronchus of the right middle lobe or lingula. Warmed saline in 20 to 50 ml aliquots was instilled into the segment and immediately aspirated. A maximum total of 300 ml of saline was used. After passing through gauze, the cells were centrifuged at 300 X G for 10 minutes in a refrigerated centrifuge, and washed twice with HA buffer as described in Example I. C. Subsequently, the cells were preincubated with HRIF or control buffer for 10 minutes at 37°C and then challenged with HRF essentially as described in Example I except that histamine was assayed radioenzymatically according to the method of Verburg, et al., Life Sciences, 32:2855 (1983). The results of this experiment, shown in FIG. 11, demonstrated that HRIF inhibits allergic mediator release from human mast cells as well as basophils. HRF induced histamine release was 18% in experiment 1 and 38% in experiment 2.

B. HRIF in Bronchoalveolar Lavage Fluid

The present inventors have also discovered that HRIF, as well as a dialyzable low molecular weight inhibitor of histamine release is present in bronchoalveolar lavage fluid obtained from human subjects. These studies are described below.

Reagents. RPMI 1640 was obtained from GIBCO Laboratories, Grand Island, NY: human serum albumin, glutamine, histamine, f-methyl peptide (FMLP), Ficoll, Hypaque, concanavalin A (Con A), penicillin, streptomycin and agarose-coupled trypsin and chymotrypsin from Sigma Chemicals Co., St. Louis, MO; Hepes from Research Organics, Inc., Cleveland, OH; tissue culture flasks from Corning Glass Works, Corning, NY; Sephadex G-50 and G-75 super fine from Pharmacia, Inc., Piscataway, NJ; hydroxyethyl starch (HetaStarch) from American McGaw, Irvine, CA; Spectrapor dialysis tubing from Spectrum Medical Industries, Los Angeles, CA; and rabbit anti-human IgE serum was from Behring Diagnostics, Somerville, NY.

Bronchoalveolar lavage was performed on 20 normal subjects and 30 mild to moderate asthmatic volunteers. Written consent was obtained from each subject before the procedure. The selection of asthmatic patients was based upon the criteria of NIH/ATS (Goldstein RA, Hurd SS. NHLBI Workshop Summaries: summary and recommendations of a workshop on the investigative use of fiberoptic bronchoscopy and bronchoalveolar lavage in asthmatics. Am. Rev. Respir.

Dis. 1985; 132:180-2). Lavage was performed with 200 ml of sterile saline. The recovery was on average 60%. The BAL fluid was spun at 450xg for 15 min. The cell-free fluid was collected and aliquoted into small volumes and frozen at -70°C until use.

HRF and HRIF activity was assayed using undialyzed as well as dialyzed (MW cut-off 3,500) BAL fluids. In addition, samples of BAL fluid were concentrated 15-20x using Amicon Ultrafiltration Cell with a membrane of MW cut-off 5,000. Samples of each BAL fluid were tested for HRF and HRIF activities using basophils from at least three different donors. HRIF was tested on basophils from two allergic donors and one normal subject and HRF was measured using basophils from allergic and/or asthmatic patients.

Generation of HRF. Leukocytes were isolated from buffy coats obtained from normal blood bank donors. MNC were isolated as described earlier (Thueson DO, Speck LS, Lett-Brown MA, Grant JA. Histamine releasing activity (HRA). I. Production by mitogen- or antigen-stimulated human mononuclear cells. J. Immunol. 1979: 123:626-632) and pulsed with Con A (25 ug/ml) in RPMI medium for 4 h, washed twice with HBSS, resuspended in RPMI medium, and cultured for 72 h. The supernatants were harvested, and concentrated 10-20x by using an Amicon ultrafiltration chamber with YM-5 filters (mol wt cut-off 5,000).

The concentrated supernatants were applied to gel filtration columns (5 x 100 cm) containing Sephadex G-75. The fractions containing histamine releasing activity (15kD to 30kD fractions), measured as described below, were pooled, concentrated 10-fold, divided into aliquots, frozen at -70°C and used as a source of HRF.

Isolation of peripheral blood leukocytes. Venous blood from donors was anticoagulated with 10 mM EDTA and sedimented with 1.5% hydroxyethyl starch for 30 min at room temperature (Lett-Brown MA, Thueson DO, Plank DE, Langford ME, Grant JA. Histamine releasing activity. IV. Molecular heterogeneity of the activity from stimulated human thoracic duct lymphocytes. Cell. Immunol. 1984; 87:434-444.) The leukocyte-rich buffy coat was collected and washed three times in HA buffer (Hepes buffer, pH 7.4 and 0.03% human serum albumin) in a refrigerated centrifuge (4°C) at 300 x g. The washed leukocytes (0.1-1% basophils) were suspended in HACM buffer (Hepes buffer, pH 7.4, 0.03% human serum albumin, 2 mM $CaCl_2$, and 1 mM $MgCl_2$). Leukocytes from 1 ml of blood were usually used for one duplicate experiment for assaying HRF and HRIF.

Assay of HRF. Aliquots of 100 ul of BAL fluid were incubated with 100 ul of leukocyte suspension for 45 min in a shaking water bath at 37°C. Each experiment was done in duplicate. At the end of the incubation, 400 ul of HA buffer were added to each tube. The supernatants were separated from the cells by centrifugation at 600 g for 5 min at 4°C. The histamine content of the supernatants was measured using an automated fluorometric analyzer (Siraganian RP. Refinements in the automated fluorometric histamine analysis system. J. Immunol. Methods. 1975; 7:283- 90 ) Spontaneous histamine release was assessed by incubating the cells in HACM buffer alone. The total histamine content of the cells was measured by lysing the cells with 3% perchloric acid. The percentage of histamine release was calculated according to the formula:

$$\frac{[(\text{histamine in the supernatant})\times 100]}{\text{total histamine in the cells}}$$

Spontaneous histamine release from the cells was usually <5% and was subtracted from the calculated histamine release.

Assay of HRIF. Aliquots of 100 ul of BAL fluid (dialyzed or undialyzed) were preincubated with 100 ul of leukocyte suspension for 5 min at 37°C and then challenged with 100 ul of HRF-containing supernatant (or other secretagogues) for an additional 45 min at 37°C. Three sets of controls were run simultaneously:

1) cells preincubated with buffer and then challenged with HRF,
2) cells preincubated with BAL fluid and then challenged with buffer, and
3) cells preincubated with buffer and challenged with buffer.

Each experiment was done in duplicate. At the conclusion of the incubation, 300 ul of HACM buffer were added to each tube, and the supernatants were separated from the cells by centrifugation at 600 g for 5 min at 4°C. The histamine content of the supernatants was measured spectrofluorometrically. The percentage of histamine released was calculated as described above. A typical experiment was carried out according to the following protocol:

| Preincubation | Challenge |
|---|---|
| (a) leukocytes + buffer | + buffer |
| (b) leukocytes + buffer | + HRF |
| (c) leukocytes + BAL | + buffer |
| (d) leukocytes + BAL | + HRF |

The percentage of inhibition of histamine release was calculated according to the formula:

$$[(b-a)-(d-c)] \times 100/(b-a)$$

The dilution of HRF for these experiments was so chosen as to obtain a histamine release in the range of 20 to 50%.

Inhibition of histamine release by anti-IgE, allergen, Con A and FMLP. These experiments were carried out as described above except that anti-IgE, ragweed allergen, Con A and FMLP were used as agonists. The concentrations of the agonists were individualized for each basophil donor so as to get 20-50% of histamine release. The exact concentration is given in the text.

Gel filtration of BAL fluids. Chromatography was performed with a column (90 x 1.5 cm) packed with Sephadex G-50 and equilibrated with HCM buffer. The column was calibrated with blue dextran, carbonic anhydrase, cytochrome C and insulin. Flow rate was maintained at 12 ml/h at 4°C. Usually 3-4 ml of concentrated (20-40 X) BAL fluids was applied to the column. Fractions of 1 ml were collected and assayed for HRF and HRIF activity.

Treatment of basophils with lactic acid and resensitization with serum. The removal of surface IgE from the basophils was accomplished by treating the cells with lactic acid (pH 3.9) for 3.5 min according to Prusansky et al (Pruzansky JJ, Grammer LC, Patterson R, Roberts M. Dissociation of IgE from receptors on human basophils. I. Enhanced passive sensitization for histamine release. J. Immunol., 1983; 131:1949). The cells were then washed three times and then resensitized with sera from an allergic asthmatic patients and healthy subject for 45 min. After two more washes, the cells were used in the histamine release test.

Statistical analysis. Results are expressed as mean ± SEM. Data were analyzed for differences using the computer program Epistat.

HRIF activity. Basophils from two allergic donors and one normal subject were used to assay the HRIF activity. Of 43 subjects studied, unconcentrated BAL fluids from 10 normal subjects and 22 asthmatic patients demonstrated moderate to strong inhibitory activity against HRF-induced histamine release from basophils obtained from all three donors (Figure 12). As described later, BAL fluids, which showed significant inhibitory activity (>15%), did not show any HRF activity when tested unconcentrated.

In a next step, 25 BAL fluids (13 asthmatic patients and 12 normal subjects) were dialyzed against HCM buffer overnight using Spectrapor dialysis membranes (MW cut-off 3,500). After dialysis, the inhibitory activity against HRF was retained in 10 samples from normal subjects (83%) and 2 samples from asthmatic patients (15%) (Figure 13). Dialysis of the dialyzed BAL fluids for shorter periods (4-8hr) has the same affect. The inhibitory activity of the BAL fluid is dose-dependent as shown in figure 14.

The inhibitory activity of the BAL fluids towards other agonists was also evaluated. Basophils were preincubated with samples of BAL fluids and then challenged with anti-IgE, concanavalin A, and FMLP. As shown in Figure 15, the undialyzed BAL fluids inhibited histamine release caused by these agonists. The inhibitory activity against anti-IgE, Con A, and FMLP was, however, totally lost in all samples after dialysis. This suggested that BAL fluids contain at least two inhibitors of histamine release, a dialyzable inhibitor which blocks histamine release caused by most secretagogues and a non-dialyzable (MW >3,500) antagonist of HRF. The latter antagonist is present in the BAL fluid from most of the normal subjects.

The approximate molecular weight of the non-dialyzable inhibitor was estimated by gel filtration chromatography (Figure 16). After concentrating 10-20x, samples of BAL fluids were applied to a column containing Sephadex G-50. The inhibitory activity was eluted from the column in the fractions which corresponded to the MW of 8kD. Thus this inhibitor resembles the MNC-derived HRIF in its molecular weight and specificity (antagonizes HRF only).

HRF activity. All unconcentrated BAL fluids were initially tested for HRF activity using basophils from two allergic patients and one normal subject. Only three BAL fluids from asthmatic patients (32% 10% and 8% of histamine release) and none from the normals released small amounts of histamine. This activity was not lost after overnight dialysis (MW>3,500). In a next step, the inventors concentrated the BAL fluids from 7 donors 10-40x using Amicon Ultrafiltration Cells and membranes (MW cut-off 5,000) and then screened against basophils from asthmatic donors. All concentrated samples showed significant histamine release as shown in the Table below.

Histamine release from basophils by BAL fluid

| BAL # | Concentration | % histamine release from basophils |
|-------|---------------|-------------------------------------|
| 1 | 10x | 34 |
| 3 | 10x | 13 |
| 5 | 20x | 55 |
| 35 | 55x | 49 |
| 39 | 28x | 40 |
| 40 | 20x | 21 |
| 43 | 36x | 73 |

Samples of BAL fluids were concentrated (MW cut-off 500) and dialyzed (MW cut-off 3,500) and then tested for histamine releasing activity using basophils from an allergic asthmatic donor. All BAL samples were obtained from normal subjects.

Effect of lactic acid treatment on HRF-induced histamine release. Previous studies have demonstrated that the surface-bound IgE from the basophils can be removed by treatment with lactic acid for 3.5 min (Pruzansky JJ, Grammer LC, Patterson R, Roberts M. Dissociatlon or IgEfrom receptors on human basophils. I. Enhanced passive sensitization for histamine release. J. Immunol., 1983; 131:1949-). BAL-HRF has been shown to be dependent an the presence or specific subtype of IgE (MacDonald SM, Lichtenstein LM, Proud D, Plaut M, Naclerio RM, MacGlashan DW, Kagey-Sobotka A. Studies of IgEdependent histamine releasing factors: heterogeneity of IgE. J. Immunol. 1987; 139:506-521.) To explore this mechanism of action of HRF, the inventors treated basophils from an allergic asthmatic patient with lactic acid and then resensitized them with serum from an allergic asthmatic or normal subject. As shown in Figure 17, HRF-induced histamine release significantly decreased after treatment with lactic acid, and this was only partially restored after resensitization with serum from an asthmatic patient but not a healthy subject. In subsequent experiments, basophils from a skin-test-positive asymptomatic subject were rendered increased responsiveness upon resensitization with serum from an allergic asthmatic patient. (FIG. 7C).

These results demonstrate that BAL fluids contain two inhibitors of histamine release from basophils, a non-specific inhibitor that blocks histamine release by most secretagogues, and HRIF, a specific antagonist of HRF. HRIF has been characterized as described above. The non-specific inhibitor has a molecular weight less than 3,500 and is lost after dialysis. Although inhibitor is present in most BAL fluids, its physiological significance is not completely understood at the present time.

The presence of HRF-and HRIF-like activities in the body fluids suggests that these cytokines may play an important role in the local regulation of basophils and perhaps mast cells. The detection of HRIF-like activity in the BAL fluid suggests that this inhibitor may represent a local control mechanism of allergic inflammation. A fine balance between HRF and HRIF may exist in body fluids under normal condition and a loss of this balance in favor of HRF may predispose to asthma and other allergic diseases.

EXAMPLE V

DEVELOPMENT OF HRIF-PRODUCING CELL LINES

The following example is designed to illustrate various procedures used by the inventors to produce cell lines that produce HRIF.

A. Generation Of Cell Lines Producing HRIF, Designated A-MLB And A-RA

Human mononuclear cells were obtained from allergic donors who had been receiving conventional immunotherapy with allergenic extracts. These patients had been judged by their physicians to have received a beneficial response to this therapy, with some reduction in clinical symptoms of allergy. Approximately 50 ml of blood was drawn and anticoagulated in heparin 10 units per ml. The mononuclear cell fraction was obtained by centrifugation over Ficoll-Hypaque (1.077 specific gravity). Approximately $60 \times 10^6$ cells were obtained, washed three times in Hanks' balanced salt solution (HBSS) and suspended at a concentration of $1 \times 10^6$/ml in RPMI 1640 with 5% heat-inactivated fetal calf serum (FCS, Hyclone, Inc., Logan, UT), 10 mM Hepes buffer, pH 7.4, 4 mM glutamine, $5 \times 10^{-6}$ M 2-mercaptoethanol, 100 units/ml penicillin and 100 ug/ml streptomycin. MNC were cultured in Corning 25 cm² tissue culture flasks in the presence of dust mite antigen (Dermatophagoides farinae $10^{-5}$ w/v, Hollister, Stier Laboratories, Spokane, WA) for 3 days. Supernatants were collected, centrifuged at $400 \times G$ at 4°C., and cells were resuspended in the same medium with addition of 20% T cell growth factor (Cellular Products, Inc., Buffalo, NY), and returned to the tissue culture flask. After the second week, half of the culture volume was removed, and the cells were pelleted by centrifugation and resuspended in fresh culture medium with added dust mite antigen ($10^{-5}$ w/v) and T cell growth factor (20% final concentration) and returned to the flask for another week. Subsequently, the cells were maintained in complete medium (RPMI with 5% FCS, 10 mM Hepes buffer, pH 7.4, 4 mM glutamine, $5 \times 10^{-6}$ M 2-mercaptoethanol, 100 units/ml penicillin and 100 ug/ml streptomycin, 20% T cell growth factor and $10^4$/ml mitomycin-treated heterologous feeder cells). Each week half the culture volume was removed and the cells were resuspended in complete medium and returned to the flask.

Heterologous feeder cells were prepared by isolating MNC from a normal donor and treating them at $5 \times 10^6$ cells/ml with mitomycin 50 ug/ml for 30 minutes at 37°C. Cells were then washed four times in HBSS, and resuspended in RPMI 1640 containing 25% FCS and 5% dimethylsulfoxide. Cells were aliquoted and frozen at -80°C. Before use, cells were washed four times in HBSS, and then suspended In RPMI 1640.

Culture supernatants were removed after 27 and 21 days for lines A-MLB and A-RA respectively, dialyzed as described above, and tested for inhibition of HRF-induced histamine release as described earlier. The results of this experiment, shown in FIG. 18, demonstrate that the two cell lines developed by this procedure produced a significant amount of HRIF. The modest inhibitory effect of conditioned medium (RPMI with 5% fetal calf serum, 10 mM Hepes buffer, pH 7.4, 4 mM glutamine, $5 \times 10^{-6}$ M 2-mercaptoethanol, 100 units/ml penicillin and 100 ug/ml streptomycin, 20% T cell growth factor) is also shown. Control histamine release was twenty percent. Unfortunately, these cell lines were lost to contamination. However, these experiments provided the basis for a preferred method described below.

## B. Generation Of Cell Line Producing HRIF, Designated JW-RW

Mononuclear cells from a patient on immunotherapy with ragweed pollen were isolated as described above and resuspended at $5 \times 10^7$ per 10 ml in RPMI plus Hepes, glutamine, 2-mercaptoethanol, penicillin and streptomycin (as described above) plus 15% FCS, and stimulated with ragweed antigen (mixed giant, short and western ragweed plus marsh elder pollens from Greer Laboratories, Lenoir, NC at $10^{-7}$ w/v) for two weeks at 37°C. in Corning 25 square cm tissue culture flasks. Then half the medium was removed, centrifuged at $300 \times g$ for 10 minutes at room temperature, and cells resuspended in the same volume of fresh RPMI-1640 medium with recombinant interleukin 2, 10 units per ml (rIL-2 from Cellular Products, Inc.). Additionally, $10^5$ mitomycin-treated heterologous cells (prepared as described above except that they were stored frozen in 50% FCS) were added as feeder cells and the cell suspension was added back to the same flask.

After the third week of culture, half the medium was removed again, cells centrifuged, resuspended in fresh RPMI plus Hepes, glutamine, 2-mercaptoethanol, penicillin, streptomycin, ragweed pollen $10^{-7}$ w/v, rIL-2 10 units per ml, 15% FCS, and $10^4$ per ml feeder cells, added back to the flask, and cultured for the fourth week. Subsequently the cells were maintained in RPMI plus Hepes, glutamine, 2-mercaptoethanol, penicillin, streptomycin, FCS, rIL-2, and feeder cells as described above freshly added weekly except that during the sixth week ragweed pollen was added for a third exposure.

This cell line produced significant amounts of HRIF but no HRF when assayed according to the procedure described in Example I E. above (FIG. 19). Although proliferation of these cells is modest, viability as well as the capacity to synthesize HRIF has been documented for 110 days. Supernatants were tested at a final dilution of 1:6. A typical dose response curve for supernatant is shown at the right of FIG. 19.

## C. Generation of Cell lines Producing HRIF, Designated WP-RW

Mononuclear cells from another allergic patient on maintenance ragweed immunotherapy were obtained from 50 ml of blood as described above. A T-enriched population was obtained by rosetting with AET-treated sheep red blood cells according to the method of Pellegrino, et al., Clin. Immunol. Immunopath., 3:324 (1975). Isolated T cells were incubated at a concentration $5 \times 10^6$/ml in a ragweed antigen-coated Petri dish for 2 hours to separate antigen binding T cells according to the method of Taniguchi and Miller, J. Exp. Med., 146:1450 (1977). The unbound cells were removed from the dish carefully and the antigen-bound cells were isolated by repeated pipetting. Antigen-binding cells were cultured at a concentration of $1 \times 10^6$/ml in RPMI 1640 containing 10% FCS and ragweed antigen $10^{-7}$ w/v for 7 days. From the second week on, the same protocol was followed as described above for JW-RW.

This procedure that utilizes enriched antigen-binding T cells also produced substantial quantities of HRIF, but no HRF, as depicted in FIG. 20. These experiments demonstrate the HRIF is synthesized by cultured antigen-binding T cells.

EXAMPLE VI

CLINICAL APPLICATIONS

Due to precautions necessarily attendant to development of every new pharmaceutical, the histamine release inhibitory factor of the present invention has not yet been tested in a clinical setting in human subjects. Therefore, the in vitro activity of this factor in inhibiting histamine release has been used to demonstrate the utility of the present invention as a pharmacologic agent since the histamine release assay is accepted by those of skill in the art as a reliable correlate of in vivo histamine release. The following prophetic embodiments represent the best mode contemplated by the present inventors for carrying out the practice of the invention in various clinical settings.

First, it is believed that the histamine release inhibitory factor will prove to be useful in treating numerous diseases in which mast cells and basophils are involved, especially the allergic disorders. In particular, these include, but are not limited to, bronchial asthma, allergic rhinitis, conjunctivitis, and urticaria. Although the best mode of administering the factor will depend on the particular clinical situation, it is believed that the factor may be most easily administered by formulating it together with a suitable pharmaceutical excipient and administering the formulation topically. For example, the factor could be formulated as a component of an aerosol for intranasal or intrabronchial administration. These delivery devices might be modified to be powered by freon. This mode of administration may be particularly useful in treating certain allergic diseases, for example, allergic rhinitis and bronchial asthma. The factor could also be administered topically to the skin or eye; this formulation might prove effective for allergic disorders at these sites. Alternatively, the factor could be formulated for intravenous, intramuscular, subcutaneous, intradermal, or intraarticular injection; such injections might be used to treat inflammatory reactions at these sites in which the triggering of mediator release from basophils and mast cells is involved in the pathogenesis of the illness. In all these formulations, suitable excipients, for example, saline or physiologic buffers, are known to those of skill in the art and may be used. Of course, in some cases, it may be desirable to incorporate a preservative into this excipient. Methods for incorporating therapeutic agents into pharmaceutical vehicles are believed to be well within the skill of the art.

As stated above, the invention has not yet been used in clinical settings. The inventors have relied upon the published results with other cytokines in predicting the acceptable pharmaceutical dosage for HRIF. The most relevant studies of an aerosol were the use of intranasal alpha 2-interferon to prevent viral upper respiratory infections. Douglas, et al., (New Engl. J. Med., 314:65 (1986)) and Hayden, et al., (New Engl. J. Med., 314:71 (1986)) administered $5 \times 10^6$ international units (IU) per day for an effective response. This cytokine is currently licensed for treatment of hairy cell leukemia at a dose of $3 \times 10^6$ IU per day by intramuscular or subcutaneous administration. Nathan, et al., (New Engl. J. Med., 315:6 (1986)) administered 20,000 to 200,000 U of interferon-gamma intradermally to persons with lepromatous leprosy for a therapeutic response. The development and clinical use of interferons has recently been reviewed by Baron, et al., The Interferon System: A Current Review to 1987, University of Texas Press, Austin. As reported in New Engl. J. Med., 313:1485 (1985), interleukin-2 has been administered intravenously to patients with cancer at doses of $10^4$ to $10^5$ units per kg over eight hours and maximal dose of up to $3.3 \times 10^6$ per kg. Finally, Vakhan-Raj, et al., (New Engl. J. Med., 317:1545 (1987)) recently injected G/M CSF by continuous infusion at doses of 1.5 to $25 \times 10^6$ units/$M^2$ of body surface for an effective response in patients with myelodysplasia. Therefore, the inventors would propose that the effective dose of HRIF will be from about $10^4$ to about $10^7$ units. Furthermore, the inventors would define a unit in the traditional manner: 1 unit causes 50% inhibition of near maximal histamine release from HRF-stimulated basophils. The exact doses of HRIF to be used in a particular clinical application must be determined by accepted pharmaceutical methods known to those skilled in the pharmaceutical arts.

Furthermore, it is also contemplated that the factor of the present invention may be used to monitor the progress of patients treated with immunotherapy. As indicated above, the present inventors have provided evidence suggesting that immunotherapy may preferentially induce synthesis of the factor and that clinical improvement of allergic symptoms correlates with increased factor synthesis. Therefore, to monitor HRIF synthesis by patient cells, patient cells may be removed and cultured to produce HRIF as described above. The HRIF activity produced by the patient cells could then be compared to a standard preparation of the factor in order to determine whether patient cells are activated to produce the inhibitory factor.

The foregoing description of the invention has been directed to particular preferred embodiments in accordance with the requirements of the patent statutes and for purposes of explanation and illustration. It will be apparent, however, to those skilled in the art that many modifications and changes may be made without departing from the scope and the spirit of the invention.

For example, numerous methods for purifying the factor may be used. In addition, in the future, the gene encoding the anti-allergic cytokine may be identified and cloned into a recombinant vector and expressed to produce a recombinant cytokine having the desired activity. Furthermore, the structural determinants critical for HRIF activity may be determined and synthetic peptides having HRIF function prepared. In addition, it is also likely that other methods for producing cell lines that produce the cytokine may be devised. For example, the cell lines described above may be fused with a continuously growing transformed cell line, for example, the cell line CEM, to produce a continuously growing hybrid cell line.

**Claims**

1. A therapeutic composition comprising a pharmaceutically acceptable carrier and a substantially purified human histamine release inhibitory factor having the following characteristics:

    (a) a molecular weight of about 8.000-10.000 daltons as determined by gel exclusion chromatography;

    (b) lability upon treatment with trypsin or chymotrypsin;

    (c) stability when heated at 60 °C for one hour; and

    (d) stability upon treatment with neuraminidase.

2. A therapeutic composition comprising a pharmaceutically acceptable carrier and a substantially purified proteinaceous human histamine release inhibitory factor, said factor exhibiting biologic properties which include inhibition of allergic mediator release from human basophils or mast cells triggered with histamine releasing factor.

3. The composition of claim 2 wherein the allergic mediator is histamine.

4. The composition of claim 1 or claim 2 wherein said factor is obtained by a process comprising:

    (a) preparing a human mononuclear cell conditioned supernatant;

    (b) fractionating the conditioned supernatant to provide a fraction which includes the factor in substantially purified form; and

    (c) collecting the fraction.

5. The composition of claim 4 wherein step (a) comprises:

    (a) obtaining a mononuclear fraction of leukocytes from an individual;

    (b) culturing the mononuclear cell fraction in an appropriate medium to produce a conditioned supernatant; and

    (c) separating the supernatant from the cultured cells.

6. The composition of claim 4 wherein said supernatant is fractionated into protein fractions of selected molecular weight ranges to provide a fraction which includes the factor in substantially purified form.

7. The composition of claim 4 wherein said fraction is selected by assaying fractions for histamine release inhibitory factor using human mast cells or human basophils triggered with histamine release factor.

8. The composition of claim 4 wherein the mononuclear cells are cultured in a tissue culture medium containing histamine.

9. A therapeutic composition comprising a pharmaceutically acceptable carrier and a substantially purified proteinaceous human histamine release inhibitory factor, said factor exhibiting biologic properties which include the following:

    (a) an ability to inhibit histamine release from human histamine containing proallergic cells;

    (b) a molecular weight of approximately 8.000-10.000 daltons as determined by gel exclusion chromatography;

    (c) lability of histamine release inhibitory activity upon treatment with trypsin or chymotrypsin;

    (d) stability of histamine release inhibitory activity upon heating to 60 °C for one hour; and

    (e) stability of histamine release inhibitory activity upon treatment with neuraminidase.

10. The therapeutic preparation of any of claims 1, 2 and 9 for use in a method for inhibiting release of an allergic mediator from human histamine containing proallergic cells comprising exposing such cells to said composition.

11. The therapeutic preparation of claim 10, wherein the allergic mediator is histamine.

12. The therapeutic preparation of any of claims 1, 2 and 9 for use in a method for treating allergic individuals comprising administering said preparation to such individuals.

13. The therapeutic preparation of any of claims 1, 2 and 9 for use in a method for treating individuals suffering from mast cell or basophil dependent disorders comprising administering said preparation to such individuals.

14. Human histamine release inhibitory factor as defined in any of claims 1, 2, or 9 for use in a method for inhibiting release of allergic mediators from human basophils or human mast cells comprising exposing said cells to said factor.

15. A method for preparing the human histamine release inhibitory factor contained in the therapeutic composition of any of claims 1, 2, or 9 comprising:

   (a) obtaining a mononuclear cells fraction of leukocytes from an individual;

   (b) culturing the mononuclear cell fraction in a suitable culture medium to produce a conditioned supernatant;

   (c) assaying the supernatant for histamine release inhibitory activity;

   (d) selecting a supernatant having histamine release inhibitory factor; and

   (e) purifying said factor from said supernatant.

16. The method of claim 15, wherein the mononuclear cell fraction is cultured in a suitable culture medium containing histamine to produce a conditioned supernatant, and wherein the histamine content is substantially reduced before the supernatant is assayed.

17. The method of claim 15 or 16 wherein the leukocytes are obtained from peripheral blood.

18. The method of claim 16 wherein the histamine is present at a concentration of $10^{-6}$ M to $10^{-10}$ M.

19. A method for producing a human histamine inhibitory factor producing cell line comprising:

   (a) obtaining a mononuclear cell fraction of leukocytes from an individual;

   (b) culturing the mononuclear cell fraction in a suitable tissue culture medium containing an antigen;

   (c) maintaining the cultured cells in a culture medium with interleukin 2 and feeder cells; and

   (d) assaying the cultured cells for the ability to produce histamine release inhibitory factor.

20. The method of claim 19 wherein the leukocytes are obtained from peripheral blood.

21. The method of claim 19 wherein the leukocytes are obtained from an individual undergoing immunotherapy and the antigen corresponds to an antigenic component of the immunotherapeutic preparation.

22. A clinical assay adapted for monitoring or diagnosing individuals suspected of having an allergic disease which comprises:

   (a) obtaining a mononuclear cell fraction of leukocytes from an individual;

   (b) culturing the mononuclear cell fraction in a suitable culture medium to produce a conditioned supernatant; and

   (c) assaying the supernatant for human histamine release inhibitory activity.

23. The assay of claim 22 wherein step (c) further comprises:

    (a) dividing a cell suspension containing human mast cells or human basophils into at least two portions;

    (b) exposing the cells in the first portion to the conditioned supernatant;

    (c) exposing the cells in both portions to a composition containing histamine releasing factor;

    (d) measuring the amount of histamine released by the cells in each portion; and

    (e) comparing the amount of histamine released by the cells in the first portion to the amount of histamine released by cells in the second portion.

24. A clinical assay adapted for monitoring or diagnosing individuals suspected of having an allergic disease which comprises:

    (a) obtaining a biological fluid from an individual; and

    (b) assaying the fluid for human histamine release inhibitory activity.

25. The assay of claim 24 wherein step (b) further comprises:

    (a) dividing a cell suspension containing human mast cells or human basophils into at least two portions;

    (b) exposing the cells in the first portion to the biological fluid;

    (c) exposing the cells in both portions to a composition containing histamine releasing factor;

    (d) measuring the amount of histamine released by the cells in each portion; and

    (e) comparing the amount of histamine released by the cells in the first portion to the amount of histamine released by cells in the second portion.

26. A substantially purified human protein factor capable of inhibiting histamine release from human proallergic cells triggered with histamine releasing factor, said protein factor having a molecular weight from 20,000 to 30,000 as determined by gel exclusion chromatography.

**Patentansprüche**

1. Therapeutische Zusammensetzung, umfassend einen pharmazeutisch verträglichen Träger und einen in wesentlichem Umfang gereinigten humanen Histamin-Freisetzungs-Inhibitorfaktor mit folgenden Eigenschaften:

    (a) Molekulargewicht von etwa 8000-10000 Dalton, bestimmt durch Gelausschlußchromatographie;
    (b) Labilität bei Behandlung mit Trypsin oder Chymotrypsin;
    (c) Stabilität bei 1-stündiger Wärmebehandlung bei 60°C; und
    (d) Stabilität bei Behandlung mit Neuraminidase.

2. Therapeutische Zusammensetzung, umfassend einen pharmazeutisch verträglichen Träger und einen in wesentlichem Umfang gereinigten, proteinartigen, humanen Histamin-Freisetzungs-Inhibitorfaktor, wobei dieser Faktor biologische Eigenschaften aufweist, die eine Hemmung der allergischen Mediator-Freisetzung aus mit Histamin-Freisetzungsfaktor getriggerten, humanen Basophilen oder Mastzellen umfaßt.

3. Zusammensetzung nach Anspruch 2, wobei es sich beim allergischen Mediator um Histamin handelt.

4. Zusammensetzung nach Anspruch 1 oder 2, wobei der Faktor durch ein Verfahren erhalten worden ist, das folgendes umfaßt:

    (a) Herstellen eines konditionierten Überstands von humanen mononuklearen Zellen;

(b) Fraktionieren des konditionierten Überstands zur Bereitstellung einer Fraktion, die den Faktor in in wesentlichem Umfang gereinigter Form enthält; und

(c) Gewinnen der Fraktion.

5.  Zusammensetzung nach Anspruch 4, wobei die Stufe (a) folgendes umfaßt:

(a) Gewinnen einer mononuklearen Fraktion von Leukozyten einer Person;
(b) Züchten der mononuklearen Zellfraktion in einem geeigneten Medium unter Bildung eines konditionierten Überstands; und
(c) Antrennen des Überstands von den gezüchteten Zellen.

6.  Zusammensetzung nach Anspruch 4, wobei der Überstand in Proteinfraktionen von ausgewählten Molekulargewichtsbereichen fraktioniert wird, um eine Fraktion bereitzustellen, die den Faktor in einer in wesentlichem Umfang gereinigten Form enthält.

7.  Zusammensetzung nach Anspruch 4, wobei die Fraktion ausgewählt wird, indem man die Fraktionen auf den Histamin-Freisetzungs-Inhibitorfaktor unter Verwendung von mit Histamin-Freisetzungsfaktor getriggerten humanen Mastzellen oder humanen Basophilen testet.

8.  Zusammensetzung nach Anspruch 4, wobei die mononuklearen Zellen in einem Gewebekulturmedium mit einem Gehalt an Histamin gezüchtet werden.

9.  Therapeutische Zusammensetzung, enthaltend einen pharmazeutisch verträglichen Träger und einen in wesentlichem Umfang gereinigten proteinartigen humanen Histamin-Freisetzungs-Inhibitorfaktor, wobei der Faktor biologische Eigenschaften aufweist, zu denen folgende Eigenschaften gehören:

(a) Fähigkeit zur Hemmung der Histamin-Freisetzung aus humanen, Histamin enthaltenden, proallergischen Zellen;
(b) Molekulargewicht von etwa 8000-10 000 Dalton, bestimmt durch Gelausschlußchromatographie;
(c) Labilität der Histamin-Freisetzungs-Inhibitoraktivität bei Behandlung mit Trypsin oder Chymotrypsin;
(d) Stabilität der Histamin-Freisetzungs-Inhibitoraktivität bei 1-stündiger Wärmebehandlung bei 60°C; und
(e) Stabilität der Histamin-Freisetzungs-Inhibitoraktivität bei Behandlung mit Neuraminidase.

10.  Therapeutisches Präparat nach einem der Ansprüche 1, 2 und 9 zur Verwendung in einem Verfahren zur Hemmung der Freisetzung eines allergischen Mediators aus humanen, Histamin enthaltenden, proallergischen Zellen, bei dem derartige Zellen einer Exposition mit dieser Zusammensetzung unterzogen werden.

11.  Therapeutisches Präparat nach Anspruch 10, wobei es sich beim allergischen Mediator um Histamin handelt.

12.  Therapeutisches Präparat nach einem der Ansprüche 1, 2 und 9 zur Verwendung in einem Verfahren zur Behandlung von allergischen Personen, umfassend die Verabreichung des Präparats an die Personen.

13.  Therapeutisches Präparat nach einem der Ansprüche 1, 2 und 9 zur Verwendung in einem Verfahren zur Behandlung von Personen, die an von Mastzellen oder Basophilen abhängigen Störungen leiden, umfassend die Verabreichung des Präparats an die Personen.

14.  Humaner Histamin-Freisetzungs-Inhibitorfaktor gemäß der Definition in einem der Ansprüche 1, 2 oder 9 zur Verwendung in einem Verfahren zur Hemmung der Freisetzung von allergischen Mediatoren aus humanen Basophilen oder humanen Mastzellen, bei dem derartige Zellen einer Exposition mit diesem Faktor unterzogen werden.

15.  Verfahren zur Herstellung des humanen Histamin-Freisetzungs-Inhibitorfaktors, der in der therapeutischen Zusammensetzung enthalten ist, nach einem der Ansprüche 1, 2 oder 9, umfassend:

a) Gewinnen einer mononuklearen Zellfraktion von Leukozyten einer Person;
(b) Züchten der mononuklearen Zellfraktion in einem geeigneten Kulturmedium unter Bildung eines konditionierten Überstands; und
(c) Testen des Überstands auf die Histamin-Freisetzungs-Inhibitoraktivität;
(d) Auswählen eines Überstands mit einem Histamin-Freisetzungs-Inhibitorfaktor; und
(e) Reinigen des Faktors aus dem Überstand.

16. Verfahren nach Anspruch 15, wobei die mononukleare Zellfraktion in einem geeigneten Kulturmedium mit einem Gehalt an Histamin unter Bildung eines konditionierten Überstands gezüchtet wird und wobei der Histamingehalt vor dem Testen des Überstands erheblich vermindert wird.

17. Verfahren nach Anspruch 15 oder 16, wobei die Leukozyten aus peripherem Blut erhalten werden.

18. Verfahren nach Anspruch 16, wobei das Histamin in einer Konzentration von $10^{-6}$ M bis $10^{-10}$ M enthalten ist.

19. Verfahren zur Herstellung einer humanen, den Histamin-Inhibitorfaktor bildenden Zellinie, umfassend:

(a) Gewinnen einer mononuklearen Zellfraktion von Leukozyten einer Person;
(b) Züchten der mononuklearen Zellfraktion in einem geeigneten Gewebekulturmedium, das ein Antigen enthalt;
(c) Halten der gezüchteten Zellen in einem Kulturmedium mit Interleukin-2 und Feeder-Zellen; und
(d) Testen der gezüchteten Zellen auf ihre Fähigkeit zur Erzeugung des Histamin-Freisetzungs-Inhibitorfaktors.

20. Verfahren nach Anspruch 19, wobei die Leukozyten aus peripherem Blut erhalten werden.

21. Verfahren nach Anspruch 19, wobei die Leukozyten von einer Person, die einer Immunotherapie unterliegt, erhalten werden und das Antigen einer antigenen Komponente des immunotherapeutischen Präparats entspricht.

22. Klinischer Test, der zur Überwachung oder Diagnose von Personen, bei denen ein Verdacht auf eine allergische Erkrankung besteht, geeignet ist, umfassend:

(a) Gewinnen einer mononuklearen Zellfraktion von Leukozyten einer Person;
(b) Züchten der mononuklearen Zellfraktion in einem geeigneten Medium unter Bildung eines konditionierten Überstands; und
(c) Testen des Überstands auf die humane Histamin-Freisetzungs-Inhibitoraktivität.

23. Test nach Anspruch 22, wobei die Stufe (c) ferner folgendes umfaßt:

(a) Unterteilen einer Zellsuspension mit einem Gehalt an humanen Mastzellen oder humanen Basophilen in mindestens zwei Teile;
(b) Durchführen einer Exposition der Zellen im ersten Teil mit dem konditionierten Überstand;
(c) Durchführen einer Exposition der Zellen in beiden Teilen mit einer den Histamin-Freisetzungsfaktor enthaltenden Zusammensetzung;
(d) Messen der von den Zellen in jedem Teil freigesetzten Histaminmenge; und
(e) Vergleichen der von den Zellen im ersten Teil freigesetzten Histaminmenge mit der von den Zellen im zweiten Teil freigesetzten Histaminmenge.

24. Klinischer Test, der sich zur Überwachung oder Diagnose von Personen, bei denen ein Verdacht auf eine allergische Erkrankung besteht, eignet, umfassend:

(a) Gewinnen einer biologischen Flüssigkeit von einer Person; und
(b) Testen der Flüssigkeit auf die humane Histamin-Freisetzungs-Inhibitoraktivität.

25. Test nach Anspruch 24, wobei die Stufe (b) ferner folgendes umfaßt:

(a) Unterteilen einer Zellsuspension mit einem Gehalt an humanen Mastzellen oder humanen Basophilen in mindestens zwei Teile;
(b) Durchführen einer Exposition der Zellen im ersten Teil mit der biologischen Flüssigkeit;
(c) Durchführen einer Exposition der Zellen in beiden Teilen mit einer den Histamin-Freisetzungsfaktor enthaltenden Zusammensetzung;
(d) Messen der von den Zellen in jedem Teil freigesetzten Histaminmenge; und
(e) Vergleichen der von den Zellen im ersten Teil freigesetzten Histaminmenge mit der von den Zellen im zweiten Teil freigesetzten Histaminmenge.

26. In wesentlichem Umfang gereinigter humaner Proteinfaktor, der zur Hemmung der Histamin-Freisetzung aus humanen proallergischen Zellen, die mit dem Histamin-Freisetzungsfaktor getriggert sind, befähigt ist, wobei der Proteinfaktor ein Molekulargewicht von 20 000 bis 30 000, bestimmt durch Gelausschlußchromatographie, aufweist.

## Revendications

1. Composition thérapeutique comportant un support pouvant être accepté de manière pharmaceutique et un facteur d'inhibition de la libération de l'histamine humaine fortement purifié ayant les caractéristiques suivantes :

   (a) un poids moléculaire d'environ 8 000 à 10 000 daltons comme déterminé par une chromatographie d'élimination sur gel ;
   (b) une instabilité après un traitement à l'aide de trypsine et de chymotrypsine ;
   (c) une stabilité lorsqu'elle est chauffée à 60° C pendant une heure ; et
   (d) une stabilité après un traitement à l'aide de neuraminidase.

2. Composition thérapeutique comportant un support pouvant être accepté de manière pharmaceutique et un facteur d'inhibition de la libération de l'histamine humain protéique fortement purifié, ledit facteur présentant des propriétés biologiques qui incluent l'inhibition de la libération de médiateur allergique à partir de cellules basophiles humaines ou de mastocytes déclenchés par le facteur libérant l'histamine.

3. Composition selon la revendication 2, dans laquelle le médiateur allergique est l'histamine.

4. Composition selon la revendication 1 ou la revendication 2, dans laquelle ledit facteur est obtenu par un processus comportant les étapes consistant à :

   (a) préparer un surnageant conditionné par des cellules mononucléées humaines ;
   (b) fractionner le surnageant conditionné afin de fournir une fraction qui inclut le facteur sous une forme fortement purifiée ; et
   (c) récupérer la fraction.

5. Composition selon la revendication 4, dans laquelle l'étape (a) consiste à :

   (a) obtenir une fraction mononucléée de leucocytes à partir d'un individu ;
   (b) cultiver la fraction de cellules mononucléées dans un milieu approprié afin de produire un surnageant conditionné ; et
   (c) séparer le surnageant des cellules en culture.

6. Composition selon la revendication 4, dans laquelle ledit surnageant est fractionné en des fractions protéiques de gammes de poids moléculaire sélectionnées afin de fournir une fraction qui inclut le facteur sous une forme fortement purifiée.

7. Composition selon la revendication 4, dans laquelle ladite fraction est sélectionnée par évaluation des fractions en ce qui concerne le facteur d'inhibition de la libération de l'histamine en utilisant des mastocytes humains ou des cellules basophiles humaines déclenchés par le facteur libérant l'histamine.

8. Composition selon la revendication 4, dans laquelle les cellules mononucléées sont cultivées dans un milieu de culture de tissu contenant de l'histamine.

9. Composition thérapeutique comportant un support pouvant être accepté de manière pharmaceutique et un facteur d'inhibition de la libération de l'histamine humain protéique fortement purifié, ledit facteur présentant des propriétés biologiques qui incluent les suivantes :

   (a) une capacité à inhiber la libération de l'histamine à partir de cellules proallergiques contenant de l'histamine humaine ;
   (b) un poids moléculaire d'environ 8 000 à 10 000 daltons comme déterminé par une chromatographie d'exclusion sur gel ;
   (c) une instabilité de l'activité de l'inhibition de la libération de l'histamine après un traitement à l'aide de trypsine et de chymotrypsine ;
   (d) une stabilité de l'activité de l'inhibition de la libération de l'histamine après un chauffage à 60° C pendant une heure ;
   (e) une stabilité de l'activité de l'inhibition de la libération de l'histamine après un traitement à l'aide de neuraminidase.

**10.** Préparation thérapeutique selon l'une quelconque des revendications 1, 2 et 9, destinée à une utilisation dans un procédé d'inhibition de la libération d'un médiateur allergique à partir de cellules proallergiques contenant de l'histamine humaine comportant l'exposition de telles cellules à ladite composition.

**11.** Préparation thérapeutique selon la revendication 10, dans laquelle le médiateur allergique est l'histamine.

**12.** Préparation thérapeutique selon l'une quelconque des revendications 1, 2 et 9, destinée à une utilisation dans un procédé de traitement des individus allergiques comportant l'administration de ladite préparation à de tels individus.

**13.** Préparation thérapeutique selon l'une quelconque des revendications 1, 2 et 9, destinée à une utilisation dans un procédé de traitement des individus souffrant d'affections dépendant des mastocytes ou des cellules basophiles comportant l'administration de ladite préparation à de tels individus.

**14.** Facteur d'inhibition de la libération de l'histamine humaine comme défini selon l'une quelconque des revendications 1, 2, ou 9 destiné à une utilisation dans un procédé d'inhibition de la libération de médiateurs allergiques provenant de cellules basophiles humaines ou de mastocytes humains comportant l'exposition desdites cellules audit facteur.

**15.** Procédé de préparation du facteur d'inhibition de la libération de l'histamine humaine contenu dans la composition thérapeutique selon l'une quelconque des revendications 1, 2, 9 comportant les étapes consistant à :

   (a) obtenir une fraction de cellules mononucléées de leucocytes à partir d'un individu ;
   (b) cultiver la fraction de cellules mononucléées dans un milieu de culture approprié afin de produire un surnageant conditionné ;
   (c) évaluer le surnageant en ce qui concerne l'activité d'inhibition de la libération de l'histamine ;
   (d) sélectionner un surnageant ayant un facteur d'inhibition de la libération de l'histamine ; et
   (e) purifier ledit facteur à partir dudit surnageant.

**16.** Procédé selon la revendication 15, dans lequel la fraction de cellules mononucléées est cultivée dans un milieu de culture approprié contenant de l'histamine afin de produire un surnageant conditionné, et dans lequel le contenu en histamine est réduit de manière importante avant que le surnageant ne soit évalué.

**17.** Procédé selon les revendications 15 ou 16, dans lequel les leucocytes sont obtenus à partir de sang périphérique.

**18.** Procédé selon la revendication 16, dans lequel l'histamine est présente à une concentration comprise entre $10^{-6}$ M et $10^{-10}$ M.

**19.** Procédé de production d'une lignée cellulaire produisant un facteur d'inhibition de la libération de l'histamine humaine comportant les étapes consistant à :

   (a) obtenir une fraction de cellules mononucléées de leucocytes à partir d'un individu ;
   (b) cultiver la fraction de cellules mononucléées dans un milieu de culture de tissu approprié contenant un antigène ;
   (c) maintenir les cellules en culture dans un milieu de culture avec de l'interleukine 2 et des cellules nourricières ; et
   (d) évaluer les cellules en culture en ce qui concerne la capacité à produire le facteur d'inhibition de la libération de l'histamine.

**20.** Procédé selon la revendication 19, dans lequel les leucocytes sont obtenus à partir de sang périphérique.

**21.** Procédé selon la revendication 19, dans lequel les leucocytes sont obtenus à partir d'un individu subissant une immunothérapie et l'antigène correspond à un composant antigène de la préparation immunothérapeutique.

**22.** Essai clinique adapté au contrôle ou au diagnostic d'individus supposés avoir une maladie allergique qui comporte les étapes consistant à :

   (a) obtenir une fraction de cellules mononucléées de leucocytes à partir d'un individu ;
   (b) cultiver la fraction de cellules mononucléées dans un milieu de culture approprié afin de produire un surnageant conditionné ; et
   (c) évaluer le surnageant en ce qui concerne l'activité d'inhibition de la libération de l'histamine humaine.

**23.** Essai selon la revendication 22, dans lequel l'étape (c) consiste de plus à :

(a) diviser une suspension cellulaire contenant des mastocytes humains ou des cellules basophiles humaines en au moins deux parties ;
(b) exposer les cellules de la première partie au surnageant conditionné ;
(c) exposer les cellules des deux parties à une composition contenant le facteur libérant l'histamine ;
(d) mesurer la quantité d'histamine libérée par les cellules de chaque partie ; et
(e) comparer la quantité d'histamine libérée par les cellules de la première partie à la quantité d'histamine libérée par les cellules de la seconde partie.

**24.** Essai clinique adapté au contrôle ou au diagnostic d'individus supposés avoir une maladie allergique qui comporte les étapes consistant à :

(a) obtenir un fluide biologique à partir d'un individu ;et
(b) évaluer le fluide en ce qui concerne l'activité d'inhibition de la libération de l'histamine humaine.

**25.** Essai selon la revendication 24, dans lequel l'étape (b) consiste de plus à :

(a) diviser une suspension cellulaire contenant des mastocytes humains ou des cellules basophiles humaines en au moins deux parties ;
(b) exposer les cellules de la première partie au fluide biologique ;
(c) exposer les cellules des deux parties à une composition contenant le facteur libérant l'histamine ;
(d) mesurer la quantité d'histamine libérée par les cellules de chaque partie ; et
(e) comparer la quantité d'histamine libérée par les cellules de la première partie à la quantité d'histamine libérée par les cellules de la seconde partie.

**26.** Facteur protéique humain fortement purifié capable d'inhiber la libération de l'histamine à partir de cellules proallergiques humaines déclenchées par le facteur libérant l'histamine, ledit facteur protéique ayant un poids moléculaire de 20 000 à 30 000 comme déterminé par chromatographie d'exclusion sur gel.

**Fig.1**

PERCENT HISTAMINE RELEASE

SPONTANEOUS    HISTAMINE

$10^{-6}M$

**Fig.2**

PERCENT INHIBITION OF HISTAMINE RELEASE

DILUTION SUPERNATANT

1:96    1:48    1:24    1:12    1:6

EP 0 398 924 B1

Fig.4

Fig.3

PERCENT INHIBITION OF HISTAMINE
RELEASE

PREINCUBATION
10 MIN          CHALLENGE

HRIF · CELLS          HRF

HRIF · CELLS   WASH 2X   HRF

HRIF · HRF          CELLS

Fig.5

PERCENT CHANGE IN RELEASE
HRIF VS BUFFER

CELLS PREINCUBATED WITH
BUFFER OR HRIF,
THEN CHALLENGE WITH

HRF

RAGWEED

ANTi-igE

CON A

PMA

C5α

Fig.6

**Fig. 6A**

PERCENT INHIBITION OF HISTAMINE RELEASE

HRIF

γ-IFN   IL-4   IL-1   IL-2

HRIF ( DILUTION )

1:24   1:12   1:6

IL/IFN (UNITS/ml)

0   50   250   500

**Fig. 6B**

PERCENT INHIBITION OF HISTAMINE RELEASE

HRIF

TNF α   IL-2
IL-1
γ-IFN   IL-4   IL-3   GM-CSF

HRIF (DILUTION)

1:48   1:24   1:12   1:6

IL/IFN/TNF/CSF (UNITS/ml)

0   0.05   0.5   5   50   500

EP 0 398 924 B1

33

Fig.7A/B

Fig.7 C

EP 0 398 924 B1

Fig.8A

*Fig. 8C*

*Fig. 8B*

Fig. 10A
ALLERGIC PATIENTS
NO IMMUNOTHERAPY

Fig. 10B
ALLERGIC PATIENTS
ON IMMUNOTHERAPY

Fig. 10C

Fig. 11
INHIBITION BY HRIF OF BRONCHOALVEOLAR MAST CELLS HISTAMINE RELEASE

*Fig.12*

INHIBITION OF BASOPHIL HISTAMINE
RELEASE BY SUPERNATANTS FROM CELL
LINES A - RA AND A - MLB

*Fig.18*

Fig.14

PERCENT INHIBITION OF HISTAMINE RELEASE

DILUTIONS OF BAL

Fig.13

PERCENT INHIBITION OF HISTAMINE RELEASE

UNDIALYZED   DIALYZED
NORMALS

UNDIALYZED   DIALYZED
ASTHMATICS

40

Fig.15

Fig-16

Fig-17

42

Fig.19B

PERCENT INHIBITION OF HISTAMINE RELEASE

SUPERNATANT DILUTION

1:24  1:12

Fig.19A

CELL LINE JW-RW

INHIBITION OF HISTAMINE

HISTAMINE

PERCENT RELEASE

DAYS IN CULTURE

Fig.20A

CELL LINE WP-RW

INHIBITION OF
HISTAMINE

HISTAMINE

Fig.20B

EP 0 398 924 B1